# FASCICULE DE BREVET EUROPEEN

(11) **EP 2 760 844 B1**
(45) Date de publication et mention de la délivrance du brevet: **21.11.2018**
(21) Numéro de dépôt: 12775783.9
(22) Date de dépôt: 27.09.2012
(51) Int. Cl.: C07D 239/91, C07D 401/12, C07D 403/12, C07D 417/14, C07K 5/10, C07K 7/06, C12Q 1/37, G01N 33/533

(54) **SUBSTRAT DE PEPTIDASE FLUOROGENE**
FLUORGENES PEPTIDASESUBSTRAT
FLUOROGENIC PEPTIDASE SUBSTRATE

(30) Priorité: 29.09.2011 FR 1158732
(43) Date de publication de la demande: 06.08.2014
(73) Titulaire: Ecole Normale Supérieure de Lyon, 69342 Lyon Cedex 07 (FR); Centre National de la Recherche Scientifique, 75794 Paris Cedex 16 (FR)
(72) Inventeur: HASSERODT, Jens, F-69006 Lyon (FR); THORN-SESHOLD, Oliver, D-80796 Munich (DE)
(74) Mandataire: Be IP
(86) Numéro de dépôt international: PCT/FR2012/052196
(87) Numéro de publication internationale: WO 2013/045854

(56) Documents cités:
- EP-A1- 2 266 963
- US-A- 5 316 906
- US-A1- 2003 059 847
- US-A1- 2010 041 748
- US-B2- 7 626 042

## Description

L'invention concerne le domaine technique des sondes pour détecter une activité enzymatique. En particulier, l'invention concerne de nouveaux substrats fluorogènes pour détecter la présence d'une peptidase catalytiquement active et un procédé de détection utilisant de tels substrats.

Dans l'analyse d'un échantillon biologique ou chimique, la détection d'une activité peptidase (ou protéase) peut être très utile (Boonacker E. et Van Noorden C.J.F. J.Histochem.Cytochem. (2001) 49, 1473-1486). Les organismes entiers, les cellules ou les extraits cellulaires, les liquides biologiques ou les mélanges chimiques sont des exemples d'échantillons biologiques ou chimiques dans lesquels une activité peptidase peut être détectée. Les exo- et endoprotéases sont une vaste famille d'enzymes qui inclut de nombreux biomarqueurs de diverses pathologies. Elles sont impliquées aussi dans de nombreux processus cellulaires bénins et font donc l'objet d'études innombrables de la part des biologistes cellulaires. Ainsi, leur détection peut donner des informations concernant un état métabolique ou morbide particulier, par exemple.

De ce fait, une sonde capable de détecter une activité peptidase est très utile. La détection de cette activité par capture de lumière de fluorescence est un procédé bien plus sensible que la collecte du reste de lumière blanche lors d'une simple absorption par la sonde, c'est-à-dire que le seuil de détection est bien plus bas. La détection d'une émission de fluorescence est très aisée à mettre en oeuvre, de sorte que les sondes fluorescentes sont des outils très attractifs pour les sciences de la vie. Par exemple, la classe des fluorophores conduisant à un transfert de proton intramoléculaire dans un état excité, nommés ESIPT (ESIPT de l'anglais « Excited State Intramolecular Proton Transfer »), est décrite dans a) Ormson, S.M., et al. Progress in Reaction Kinetics (1994) 19, 45-91 ; et b) Legourrierec, D., et al. Progress in Reaction Kinetics (1994), 19, 211-275). La première interprétation de la fluorescence élevée trouvée dans certains composés phénoliques comme étant un phénomène ESIPT peut être attribuée à Weller (pour le salicylate de méthyle : Weller, A. (1961). Fast Reactions of Excited Molecules. Progress in Reaction Kinetics and Mechanism 1, 187), età Heller et Williams (pour les hydroxyphenylbenzoxazoles : Heller A., et Williams, D.L., J. Phys. Chem. (1970)74, 4473-4480).

La classe des fluorophores ESIPT est particulièrement attractive pour le chercheur dans les sciences de la vie, du fait de ses propriétés exceptionnelles comparées aux fluorophores conventionnels. Les propriétés exceptionnelles des fluorophores ESIPT sont :
(a) un grand déplacement de Stokes dépassant souvent 130 nm et capable d'atteindre des valeurs de 250 nm qui permet des choix instrumentaux maximisant la sensibilité de détection ;
(b) une excellente résistance au photoblanchiment avec des taux qui peuvent être d'ordres de grandeur supérieurs à ceux de fluorophores modèles comme la fluorescéine ;
(c) la possibilité de concevoir des fluorophores qui émettent une fluorescence brillante à l'état solide, une propriété rare parmi tous les fluorophores connus. Cette dernière performance permet la production d'un signal de haute intensité au site de l'activation de la sonde, avec une dilution minimale causée par la diffusion ; et enfin,
(d) la possibilité de concevoir des fluorophores phénoliques ESIPT qui émettent dans le rouge ou le proche infrarouge (600 à 850 nm) où la transparence des tissus est la plus élevée ; la sonde correspondante serait particulièrement appropriée pour l'imagerie chez l'animal vivant.

La majorité de ces propriétés si un tel fluorophore était intégré dans une sonde constituerait un apport important par rapport aux propriétés des sondes commercialisées conventionnelles qui sont entravées dans leurs performances par de petits déplacements de Stokes et un photoblanchiment moyen à élevé, et qui conduisent sans exception à des fluorophores à l'état de solution.

Dans le cadre de l'invention, les inventeurs se sont intéressés au choix judicieux d'un motif clivable par une enzyme qui confèrerait à la sonde complète, incorporant un fluorophore ESIPT comme décrit ci-dessus, une hydrosolubilité totale, condition préalable pour atteindre les sites et les tissus d'intérêt. Une telle sonde permettrait une augmentation significative de la sensibilité de détection, qui, pour sa part, permettrait une réduction de la dose et pourrait ainsi, notamment, être adaptée à une application en imagerie *in vivo,* tout en réduisant les problèmes de toxicité. Le niveau de sensibilité est étroitement lié (i) au taux de photoblanchiment, (ii) au degré d'accumulation du signal fluorescent sur son site de production (et donc au taux de diffusion depuis ce site, et à la question de savoir si le fluorophore précipite ou non) (iii) au mode éteint/allumé réel dans lequel la sonde fonctionne (absence de signal faussement positif dû à l'hydrolyse spontanée de la sonde), et (iv) au degré de superposition du spectre d'excitation et du spectre d'émission (leur séparation au niveau de la ligne de base étant la configuration la plus favorable ; voir point (a) ci-dessus). Le point (iv) revêt une importance toute particulière, car la séparation complète au niveau de la ligne de base offre l'opportunité d'un choix de filtres très large pour la source lumineuse (pour exciter la molécule à toutes les longueurs d'ondes possibles), mais de manière encore plus importante, pour le détecteur (pour récolter des photons provenant de toutes les longueurs d'ondes émises par le fluorophore). Le point (iv) minimise aussi la perturbation du processus de détection par l'autofluorescence tissulaire (caractérisée par un faible déplacement de Stokes des fluorophores naturels), un problème récurrent rencontré avec les fluorophores établis, qui présentent eux aussi un faible déplacement de Stokes.

Au cours des années récentes, il y a eu un intérêt croissant dans la conception de substrats d'enzymes à trois composants déclencheur/agent de liaison/fluorophore en utilisant des espaceurs à auto-immolation comme agent de liaison. Parmi la classe importante des fluorophores ESIPT, la 2-hydroxyphénylquinazolinone (HPQ) est particulièrement intéressante, étant donnée qu'elle est parfaitement insoluble dans les milieux aqueux/physiologiques, tout en étant fortement fluorescente à l'état solide et seulement à l'état solide. Néanmoins, il est très difficile d'utiliser la HPQ dans la conception d'une sonde moléculaire qui renseigne sur l'activité d'une acyle hydrolase (une estérase ou une peptidase). D'ailleurs, les principales activités pour lesquelles une sonde à base de HPQ a déjà été conçue (et commercialisée) sont celles de phosphatases et de glycosidases, du fait de l'impossibilité de créer une sonde à base de HPQ stable avec un hydroxyle phénolique acylé car le produit résultant est enclin à une hydrolyse spontanée rapide qui, bien entendu, libère la HPQ insoluble libre et produit ainsi un signal fluorescent erroné ("signal faussement positif'). Il convient également de noter que la commercialisation par Molecular Probes de tels composés acylés (ELF97 substrat estérase (ELF97 acetate) et ELF97 substrat lipase (ELF97 palmitate)) a été interrompue en 2005 et leur étude par certains chercheurs est restée sans suite (C. Kragelund et al. / FEMS Microbiology Ecology 54 (2005) 111-122). La raison est l'instabilité à l'hydrolyse intrinsèque des esters phénoliques, en particulier des esters phénoliques ESIPT, un fait qui est encore aggravé par l'assistance anchimérique à l'hydrolyse de la part d'un nucléophile interne (l'azote de l'imine).

Une autre solution a été envisagée par d'autres équipes pour s'affranchir de cette auto-dégradation : le passage à un fluorophore ESIPT dérivé du HPQ qui possède un groupement amino à la place d'un groupement hydroxy. Cette solution a été envisagée car il est connu que le composé acylé correspondant, qui donne une liaison carboxamide à la place d'un ester carboxylique, a des fortes chances de ne plus subir d'hydrolyse spontanée : (a) Marie Cellier, Olivier J. Fabrega, Elizabeth Fazackerley, Arthur L. James, Sylvain Orenga, John D. Perry, Vindhya L. Salwatura, Stephen P. Stanforth, Bioorganic&MedicinalChemistry 19 (2011) 2903-2910, titre : « 2-Arylbenzothiazole, benzoxazole and benzimidazolederivatives as fluorogenicsubstrates for the détection of nitroreductase andaminopeptidaseactivity in clinically important bacteria » ; (b) Fabrega, O.; James, A.; Salwatura, V. L.; Orenga, S.; Stanforth, S. P. Patent WO2008152305 ; et Fabrega, O.; James, A.; Salwatura, V. L.; Orenga, S.; Stanforth, S. P. Patent WO2008152306.

Or, cette approche présente les inconvénients suivants :
- les composés ressemblant à un fluorophore capable de montrer l'effet ESIPT et comprenant une unité aniline à la place d'une unité phénol (tel que présent dans la HPQ) ne montrent presque pas de bande ESIPT caractérisée par un grand déplacement de Stokes. Dans le meilleur des cas, ces fluorophores se comportent comme des fluorophores « normaux » avec un faible déplacement de Stokes, donc avec une longueur d'onde d'émission proche de celle d'excitation. Les auteurs suivants déclarent alors de tels composés « non-fluorescents » ou « ne montrant pas de fluorescence ESIPT » : (a) J. K. Dey ; S. K. Dogra, Bull. Chem. Soc. Jpn. 1991, 64, 3142-3152 ; (b) K. Kuldov, Y. Eichen, P. Emele, H.P. Trommsdorff, Journal of Luminescence 72-74 (1997) 513-514 . Ce phénomène d'une absence de fluorescence ESIPT des analogues « aniline » par rapport à l'HPQ a également été mis en évidence dans la gamme des « quinazolinones » par l'un des inventeurs de la présente demande de brevet : Michael Waibel, Jens Hasserodt, Tetrahedron Letters 50 (2009) 2767-2769. L'intérêt de travailler avec des quinazolinones (=> HPQ) à la place des benzothiazoles, -oxazoles ou -imidazoles repose sur l'insolubilité supérieure des quinazolinones, ce qui conduit donc à une maximisation du signal au lieu de la précipitation du fluorophore suite à sa transformation par l'enzyme cible. Cette insolubilité maximale a été attribuée au phénomène de dimérisation par l'intermédiaire de deux liaisons hydrogènes inter-moléculaires entre les unités lactames dans la maille crystalline du produit précipité (Diwu, Z.; Klaubert, D. H.; Haugland, R. P., SPIE Advances in Fluorescence Sensing Technology IV (1999), 3602, 265-274).
- les composés ressemblant à un fluorophore capable de montrer l'effet ESIPT et comprenant une unité aniline (2-aminophényle) dont le groupement amino est acylé (S. Santra et al.,J. Phys. Chem. A 2000, 104, 476-482) ou sulfonylé (Christoph J. Fahrni et al., J. Phys. Chem. A 2002, 106, 7655-7663), et qui comportent donc un groupement électro-attracteur et possèdent toujours un atome d'hydrogène sur l'atome d'azote, montrent une fluorescence ESIPT avec une intensité élevée par rapport au dérivé non-acylé. Une sonde qui possède un tel groupement et qui cible une peptidase devrait ainsi montrer plutôt un mode de fonctionnement « allumé => éteint », mode qui est très peu attractif car il est difficile de savoir si une baisse d'intensité du signal correspond à une conversion ou à une simple diffusion de la sonde initiale.

En 2010, l'un des inventeurs de la présente demande de brevet a également décrit dans Chem. Eur. J.(2010), 16, 792-795, un substrat d'enzyme à trois composants : séquence peptidique/espaceur/HPQ où l'espaceur inclut un groupe O,O-acétal acylé lié à la HPQ. Bien que la sonde étudiée soit relativement stable dans les conditions physiologiques, elle présente cependant un taux de dégradation résiduel conduisant à un signal fluorescent faussement positif dans des applications d'imagerie cellulaire.

Des solutions conduisant à une absence plus marquée, voire complète de dégradations spontanées des sondes et donc de production de signaux erronés, une condition préalable fondamentale pour leur utilisation dans des applications *in vitro* et *in vivo,* ont été proposées avec l'utilisation d'un espaceur para-aminobenzyle greffé sur le fluorophore phénolique (WO2008145830, Richard, J.A., et al. Bioconjugate Chemistry (2008)19, 1707-1718). Bien que de tels espaceurs para-aminobenzyle aient déjà été utilisés dans la conception de pro-médicaments depuis le début des années 1980 (Wakselman, M. New Journal of Chemistry (1983), 7, 439-447 ; Carl, P.L., et al. Journal of Medicinal Chemistry (1981)24, 479-480 ; Toki, B.E., et al. Journal of Organic Chemistry (2002)67, 1866-1872 ; et Senter, P.D., US2003096743), ils possèdent un inconvénient majeur connu : l'utilisation d'espaceurs para-aminobenzyle (ou de leurs analogues oxygénés) dans des substrats d'enzymes artificiels conduit à l'alkylation permanente de la protéine, souvent à proximité ou à l'intérieur du site catalytique. Cette propriété négative a, par la suite, été utilisée de manière avantageuse dans de nombreux articles qui ont proposé des substrats inactivant les enzymes, c'est-à-dire conduisant à une enzyme qui n'est plus capable de convertir encore les molécules de substrat, comme le montre le **Schéma 1** suivant.

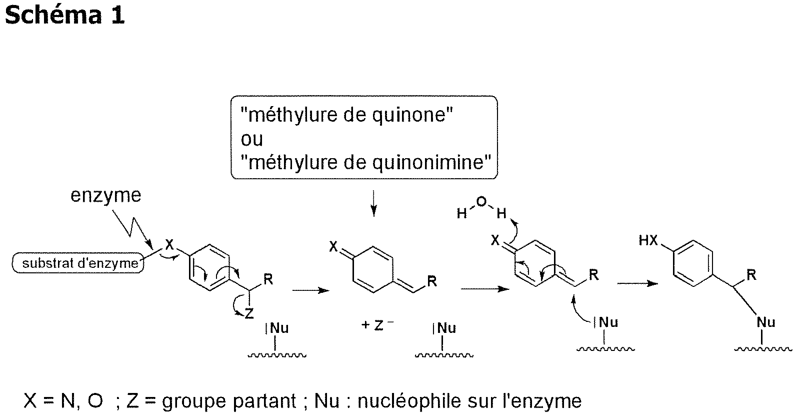

Les différents travaux sur le marquage enzymatique par des substrats d'enzyme à base d'ortho- ou para-amino- (ou hydroxy-) benzyle montrent que les méthylures de quinonimine sont des espèces très réactives qui alkylent les nucléophiles et qui risquent de modifier au hasard les propriétés moléculaires des biomacromolécules qui sont dans leur voisinage immédiat. L'activation ou les capacités catalytiques réduites de l'enzyme cible sont, bien entendu, très néfastes pour la sensibilité de les expériences d'imagerie utilisant une telle sonde fluorogène, car l'amplification enzymatique est perdue. Mis à part l'inactivation directe de l'enzyme cible, l'alkylation aléatoire de la surface de cette protéine ou de toute autre protéine voisine présente également le risque d'une réponse immunitaire. Les deux cas génèrent une tolérance limitée de la part de l'organisme respectif et donc une toxicité élevée pour celui-ci.

Dans ce contexte, la demanderesse s'est proposée d'offrir d'autres espaceurs 1) qui permettraient de créer une sonde stable incorporant un fluorophore ESIPT, avec ainsi une minimisation de la fluorescence de fond de la sonde non transformée, et 2) qui ne présenteraient pas les mêmes risques en termes de toxicité que l'espaceur aminobenzyle décrit dans l'état de la technique. La demanderesse s'est intéressée aux espaceurs diamine. Les espaceurs diamine, en tant que partie d'un carbamate phénolique tertiaire, ont été utilisés auparavant pour créer des promédicaments qui peuvent être transformés en médicament actif, suite à une transformation par une enzyme cible spécifique. Il est bien connu dans le domaine de la conception des pro-médicaments que les aminoéthylcarbamates phénoliques s'immolent à des taux appréciables, dans les conditions physiologiques, seulement si les deux amines sont secondaires, c'est-à-dire sont chacune doublement alkylées, comme le montre le **Schéma 2** ci-dessous (Saari, W.S., et al. Journal of Médicinal Chemistry (1990) 33, 97-101).

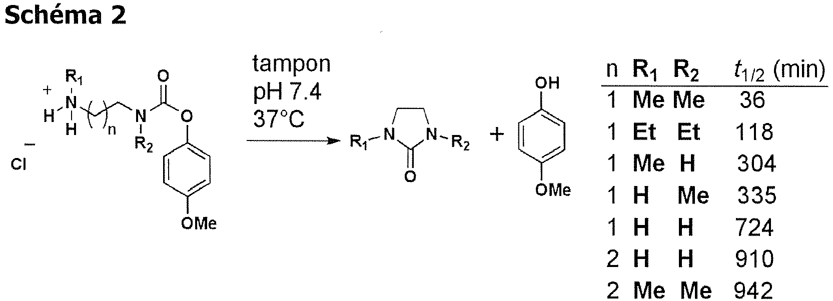

Les résultats expérimentaux décrits dans la demande WO2007/140272 sur la conception de pro-médicaments montrent clairement l'impact néfaste sur le taux d'immolation de l'espaceur, quand l'amine distale est primaire (seulement un substituant alkyle), et non secondaire. Les taux de conversion résultants sont montrés sur le **Schéma 3** suivant.

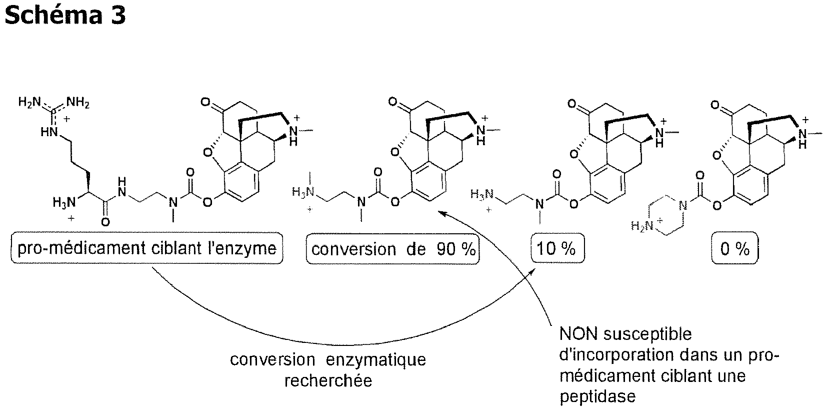

Tandis que la construction avec deux amines secondaires s'immole à raison de 90 % en phénol libre en 24 heures dans des milieux physiologiques, la construction avec une amine primaire et une amine secondaire (faisant partie d'un carbamate tertiaire) se convertit seulement à raison de 10 % dans ces conditions.

Un point important, qui est souligné dans la demande de brevet WO 2007/140272, est que l'espaceur avec deux amines secondaires (conversion de 90 % s'il est soumis à des conditions physiologiques) n'est pas utile pour l'incorporation dans un pro-médicament ciblant une peptidase car la plupart des peptidases (ou amidases) ne reconnaissent pas les liaisons peptidiques tertiaires. Ce fait établi depuis longtemps a été confirmé par une étude très récente, sur la libération de fluorophores phénolique par des sondes ciblant des peptidases profluorescentes (Meyer, Y., et al. OrgBiomolChem (2010)8, 1777-1780).

Dans ce contexte, l'objectif de l'invention est de proposer de nouveaux substrats de peptidases qui soient stables en milieu aqueux et qui restent non fluorescents ou faiblement fluorescents à une longueur d'onde bien différente de celle à laquelle le fluorophore libéré est lui fluorescent, mais qui réagissent avec des peptidases pour produire une petite molécule fluorescente basée sur ESIPT comme la HPQ. Selon l'invention, il est envisagé de proposer un substrat de peptidase ayant les propriétés suivantes
- spécificité pour une peptidase particulière,
- absence de signal faussement positif produite par la dégradation spontanée de la sonde, en particulier comparée aux sondes ayant des espaceurs O,O acétal,
- bonne cinétique d'immolation,
- biocompatibilité sans la toxicité identifiée qui est associée avec l'utilisation d'espaceurs aminobenzyle.

Plus précisément, l'invention concerne un substrat de peptidase de formule (I) : 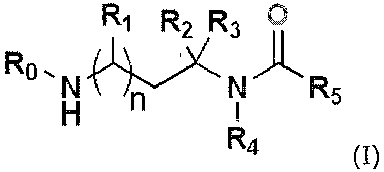 dans laquelle :
- R₀ est un groupement peptidyle ou aminoacide lié au groupement NH *via* son extrémité carboxy-terminale,
- n est égal à 0 ou 1,
- R₁ est un atome d'hydrogène ou un groupe méthyle, iso-propyle, iso-butyle ou benzyle,
- R₅ est un dérivé phénoxy de formule (A) (voir ci-dessous) dont le dérivé phénol correspondant appartient à la classe des fluorophores ESIPT,
- R₂, R₃ et R₄ sont définis comme suit :
   - soit R₂ est un (C₁-C₄)alkyle, R₃ est un (C₁-C₄)alkyle et R₄ est un (C₁-C₄)alkyle,
   - soit R₂ est un atome d'hydrogène et R₃ et R₄ sont liés entre eux et forment avec les atomes de carbone et d'azote auxquels ils sont liés un hétérocycle aliphatique,
sous la forme d'un mélange d'isomères optiques ou de diastéréoisomères selon toutes proportions, ou sous une forme enrichie en un isomère optique ou en un diastéréoisomère.

Le substrat selon l'invention agit comme une sonde moléculaire capable de révéler la présence d'une activité enzymatique spécifique par détection de fluorescence. Plus spécifiquement, la sonde est invisible avant de rencontrer l'enzyme ciblée, (c'est-à-dire "sonde furtive"), mais quand elle est modifiée chimiquement par ladite enzyme, elle se fragmente *via* une réaction en cascade pour produire une fluorescence intense. La sonde est composée de trois composants moléculaires i) un espaceur intelligent qui porte, à une extrémité, ii) un substrat de l'enzyme cible et, à l'autre, iii) un dérivé phénoxy qui, quand il est libéré par ladite fragmentation, pour donner le dérivé phénol correspondant appartient à la classe des fluorophores ESIPT. Pour surmonter la cinétique d'immolation défavorable qui est associée avec un espaceur aminoéthylcarbamate qui porte un groupe amine primaire en position distale par rapport au groupement carbamate tertiaire, la présente invention propose un groupe de nouveaux espaceurs diamines qui sont substitués de telle manière qu'ils sont pré-organisés pour la cyclisation en une urée cyclique. Cette pré-organisation accélère le processus d'immolation lors de la transformation par une enzyme. De plus, la cyclisation de l'espaceur utilisé dans le cadre de l'invention forme des urées qui sont connues pour leur grande stabilité et leur biocompatibilité générale.

Cette innovation technologique permet d'obtenir deux propriétés fondamentales pour la sonde moléculaire correspondante : (a) elle l'a rend insensible à la dégradation spontanée et donc à la production d'un signal fluorescent faussement positif, et (b) elle garantit une cinétique de fragmentation rapide lors de la transformation par l'enzyme cible pour une performance adaptée aux applications dans le domaine des sciences de la vie. Le groupe R₀ est capable d'être clivé du reste du substrat par l'action de la peptidase cible, ce qui conduit à un intermédiaire qui s'immole spontanément et rapidement pour produire un signal fluorescent.

Le précipité qui peut être obtenu à partir du substrat de peptidase selon l'invention, par clivage de la liaison covalente entre NH et R₀, suivi d'un clivage de la liaison -C(O)-R₅ suite à une cyclisation de l'espaceur, donne un signal fluorescent. Selon un mode de réalisation particulier, ce précipité est fortement fluorescent tandis que le substrat de peptidase original est peu fluorescent ou pas fluorescent du tout ; la sonde intacte fonctionne ainsi selon le mode éteint/allumé.

Selon un autre aspect, l'invention concerne un procédé pour détecter la présence d'une peptidase catalytiquement active, au moyen d'un substrat selon l'invention. Plus précisément, l'invention concerne un procédé pour détecter la présence d'une peptidase catalytiquement active comprenant les étapes de :
- mise en contact d'un échantillon suspecté de contenir ladite peptidase avec un substrat selon l'invention ;
- application de conditions appropriées pour permettre la formation d'un précipité fluorescent ; et
- analyse quantitative ou qualitative dudit précipité fluorescent.

En particulier, un tel procédé de détection peut être mis en oeuvre dans des conditions physiologiques, notamment dans un milieu aqueux tamponné à un pH de l'ordre de 7,4.

Dans un mode de réalisation de l'invention, l'analyse du précipité fluorescent comprend les étapes suivantes :
- exposition du précipité fluorescent à une source lumineuse capable de produire une lumière à une longueur d'onde d'absorption du précipité fluorescent ; et
- détection de la fluorescence résultante du précipité.

L'invention va être décrite de manière plus détaillée. Auparavant, certains termes utilisés dans la définition du substrat (I) vont être définis.

Par « alkyle », on entend une chaîne hydrocarbonée saturée qui peut être linéaire ou ramifiée. Méthyle, éthyle, n-propyle, isopropyle, iso-butyle et tert-butyle sont des exemples de groupes (C₁-C₄)alkyle (alkyle avec 1 à 4 atomes de carbone).

Par « aryle », on entend un groupe phényle, naphtyle ou cinnamyle.

Par groupe « peptidyle », on entend un enchaînement d'au moins deux acides aminés liés entre eux par une liaison peptidique. Dans le cadre de l'invention, le ou les acides aminés présents dans R₀ peuvent être des acides aminés naturels ou non, mais seront, de préférence, choisis parmi les 20 acides aminés naturels (=protéinogéniques), éventuellement sous une forme salifiée ou protégée. La fonction N-terminale de l'acide aminé terminal pourra éventuellement être salifiée ou fonctionnalisée. A titre d'exemple de forme salifiée, on peut citer la forme chlorhydrate, tosylate ou trifluoroacétate.

Par « chaine latérale d'un acide aminé », on entend la chaine latérale des acides aminés primaires. A titre d'exemple de telle chaine latérale, on peut citer les groupes méthyle (chaine latérale de l'alanine), iso-propyle (chaine latérale de la valine), iso-butyle (chaine latérale de la leucine), benzyle (chaine latérale de la phénylalanine).

La « fluorescence » est la propriété par laquelle une molécule qui est excitée par de la lumière d'une longueur d'onde donnée émet de la lumière à une plus grande longueur d'onde. La fluorescence est un phénomène qui résulte de l'interaction d'un fluorophore avec un photon incident. Ce processus est appelé excitation. L'absorption du photon amène un électron dans le fluorophore à passer de son état fondamental à un niveau d'énergie supérieur. Ensuite, l'électron revient à son niveau original en émettant un photon. Ce processus est appelé émission de fluorescence. Le fluorophore émet ensuite de la lumière à une plus grande longueur d'onde que celle du photon absorbé. Ceci est dû simplement au fait que l'énergie du photon émis est inférieure à celle du photon absorbé, du fait de la dissipation d'énergie pendant la durée de vie de l'état excité. Cette définition est donnée dans la demande de brevet WO 2004/058787.

Les composés (I) selon l'invention sont appelés « substrat de peptidase » car ils sont transformés en une autre substance pendant une réaction chimique catalysée par une peptidase. Pendant une telle réaction, les composés (I) (appelés également « sonde ») sont clivés en un précipité fluorescent et un produit non fluorescent par l'action de la peptidase spécifique.

R₅ est un dérivé phénoxy R₅ de formule (A) 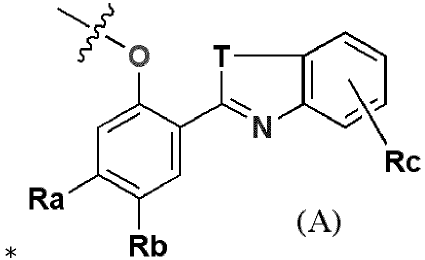 dans laquelle
- T est -NH-C(O)-, -S-, -O-, -NH, N-alkyle ou N-aryle,
- Ra est l'hydrogène ou un substituant carboné attracteur d'électrons, comme -CN ou -COORd, avec Rd qui représente un groupe (C₁-C₄)alkyle, ou bien Ra est -CONReRf, avec Re et Rf, identiques ou différents, qui représentent l'hydrogène ou un groupe (C₁-C₄)alkyle, ou bien Ra est -CF₃, ou un groupe 2-oxazolyle, 2-thiazolyle, 2-imidazolyle (benzo-condensé ou pas), 4-pyrimidinon-2-yle, ou quinazolinon-2-yle,
- Rb est l'hydrogène, un atome de chlore, -OH, -NH₂, -NRgRh ou-ORg, avec Rg et Rh identiques ou différents, qui représentent (C₁-C₄)alkyle,
- ou bien Ra et Rb sont liés entre eux pour former une chaine hydrocarbonée comprenant 4 ou 5 chainons, saturée ou insaturée, substituée ou non substituée, éventuellement interrompue par un ou plusieurs hétéroatomes choisis parmi N, S et O,
- Rc est l'hydrogène, Br, Cl, I ou F.

Lorsque la peptidase cible a libéré un groupe amino primaire libre à l'extrémité opposée au groupe carbamyle de l'espaceur, ce dernier se cyclise spontanément en libérant ainsi le phénol qui devient ainsi fortement fluorescent.

Dans un mode de réalisation particulier, le substrat de peptidase selon l'invention est de formule (IA) : 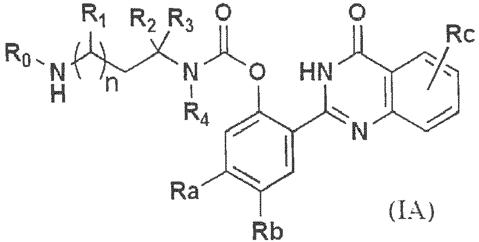 où n, R₀, R₁, R₂, R₃, R₄, Ra, Rb et Rc sont définis comme ci-dessus pour (I) et (A). Selon des modes de réalisation particulier, l'invention concerne des composés de formule (I) avec R₅ qui représente un groupe (A) ou (B) et des composés de formule (IA), Ra=Rb=Rc=H ou bien Ra=H, Rb=Rc=Cl avec Rc, de préférence, en position para par rapport à la position de l'atome d'azote. Selon d'autres modes de réalisation particulièrs, Ra et Rb sont liés entre eux, de sorte que le substrat selon l'invention répond à la formule (IA') ou (IB') : 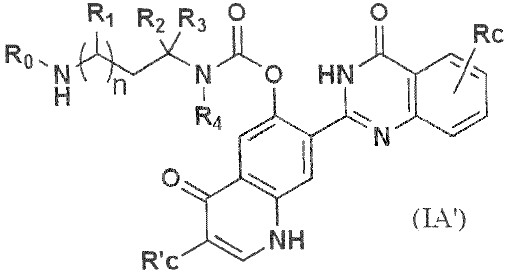 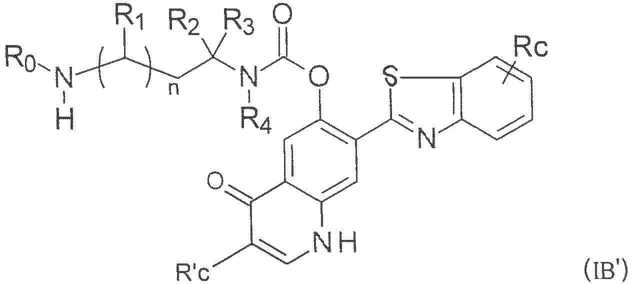 où n, R₀, R₁, R₂, R₃, R₄ et Rc sont définis comme ci-dessus pour (I) et (A) et R'c représente un atome d'hydrogène ou un groupe -COO(C₁-C₄)alkyle. Les composés dans lesquels Rc=H ou Cl en para par rapport à l'atome d'azote sont préférés.

Dans un mode de réalisation des substrats (I), (IA), (IA') et (IB') selon l'invention, R₂=H et R₃ et R₄ sont liés entre eux et forment un enchaînement -(CH₂)ₘ- avec m = 3, 4 ou 5.

Dans un autre mode de réalisation des substrats (I), (IA), (IA') et (IB') selon l'invention, R₂, R₃ et R₄, identiques ou différents, sont un (C₁-C₄)alkyle, par exemple un méthyle ou un éthyle. Par exemple, R₂=R₃=R₄=-CH₃.

Ces deux types de façon de préorganiser l'espaceur éthylènediamine pour la cyclisation en une urée cyclique, consistant soit à introduire deux substituants alkyle sur le carbone en alpha du groupement carbamate, soit à inclure la liaison entre l'azote du groupement carbamate et son carbone alpha dans un hétérocycle, accélèrent le processus d'immolation. La liaison entre l'azote du groupement carbamate et son carbone alpha est, par exemple, introduite dans un hétérocycle appartenant à la classe des pyrrolidines (à 5 chaînons) ou à celle des pipéridines (à 6 chaînons).

Dans ces différents modes de réalisation, n peut être égal à 0 ou à 1. Dans le cas où n=1, R₁ sera, de préférence, un groupe méthyle, iso-propyle, iso-butyle ou benzyle.

Le **Schéma 4** ci-après montre deux exemples de substrats fluorogènes (exemples **I.1** et **I.2**) et les cascades de réactions selon lesquelles ils se fragmentent, après initiation due au clivage par la peptidase cible dans le cas où n=0.

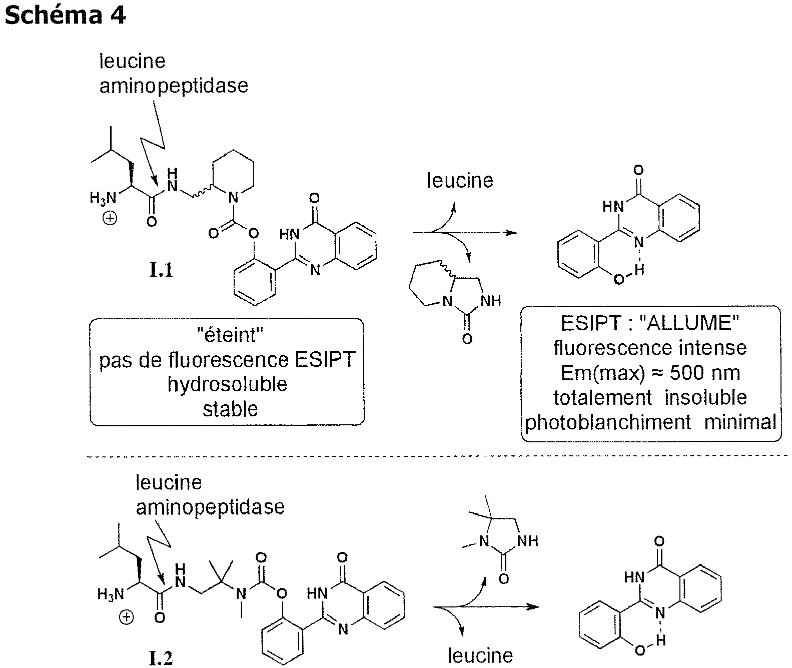

Les substrats **I.3, I.4 et I.5** suivants représentent trois autres exemples selon l'invention dans lesquels n=0.

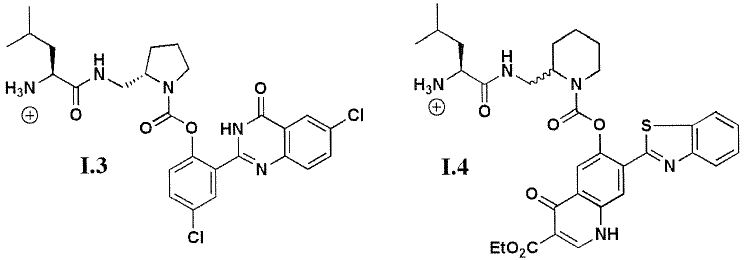

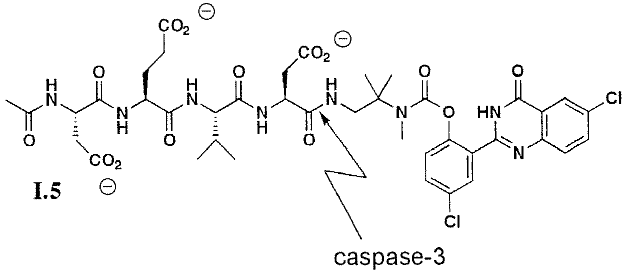

Il est également possible que n=1. Dans ce cas, R₁ sera, de préférence, différent de l'hydrogène et pourra, par exemple, représenter un groupe méthyle. Le substrat **I.6** ci-après représente un exemple selon l'invention dans lequel le groupe espaceur avec n=1 mime le chaînon latéral du 1^{er}résidu acide aminé à côté du C-terminal de la liaison peptidique scissile (la position « P1' » selon la convention de Schechter et Berger ; Schechter et Berger (1967) Biochem. Biophys. Res. Commun.27 (2): 157-162). L'intérêt de travailler avec un espaceur dans lequel n=1 selon l'invention consiste en l'augmentation de la reconnaissance moléculaire par une endoprotéase (telle que la PSA) pour la sonde qui présente également le 1^{er} chaînon latéral « post-scissile » (un méthyle pour l'alanine) de la séquence peptidique préférée de la peptidase cible, (Coombs et al. Chem. Biol. 1998, 5, 475), ceci pour augmenter la vitesse de conversion, voire même observer une transformation quelconque pour certaines endoprotéases. Il est à souligner que la présence de ce chaînon devrait procurer un avantage cinétique à l'immolation de l'espaceur, qui pourrait permettre de compenser, au moins partiellement, la perte de vitesse due au passage d'un espaceur à cinq membres (n=0) à un espaceur à six membres (n=1). De plus, le substrat **I.6** comprend un dérivé di-chloro de l'HPQ utilisé par la société Invitrogen-Molecular Probes pour sa technologie ELF 97 pour la granularité avantageuse de son précipité dans le milieu physiologique.

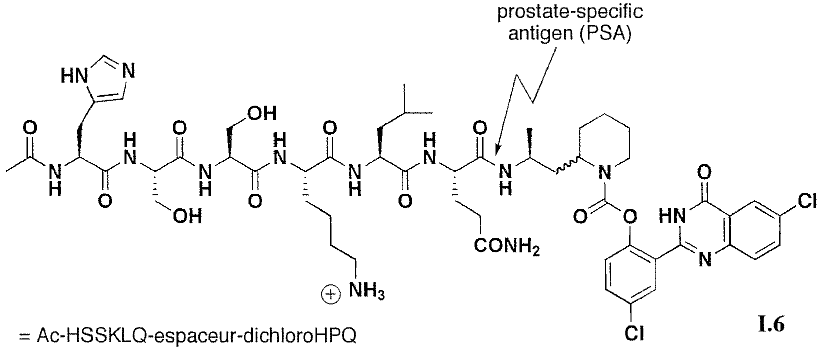

Les exemples **I.1** et **I.2** comprennent le fluorophore ESIPT classique hydroxyphényl-quinazolinone ("HPQ") (US3169129), et les exemples **I.3, I.5** et **I.6** la dichloro-HPQ, dont la longueur d'onde d'émission maximale est située entre 500 et 550 nm selon le motif de substitution. Cependant, cette invention est applicable à tous les phénols permettant d'obtenir un phénomène ESIPT, comme c'est le cas de l'exemple **I.4** car la chimie de suppression de la fluorescence ESIPT est identique et simple : l'incorporation de l'hydroxyle phénolique dans le groupement carbamate empêche la formation de la liaison hydrogène interne. Dans l'invention, le plus simple fluorophore (A) avec Ra=Rb=H peut être utilisé. Un fluorophore plus complexe avec différentes substitutions peut être utilisé. Pour leur préparation, on peut se référer à EP 0641 351 ou WO 2004/058787, par exemple.

Des exemples de fluorophores phénoliques ESIPT sont décrits dans la littérature, leurs maxima d'excitation et d'émission, ainsi que leur rendement quantique ("QY") sont énumérés ci-après. 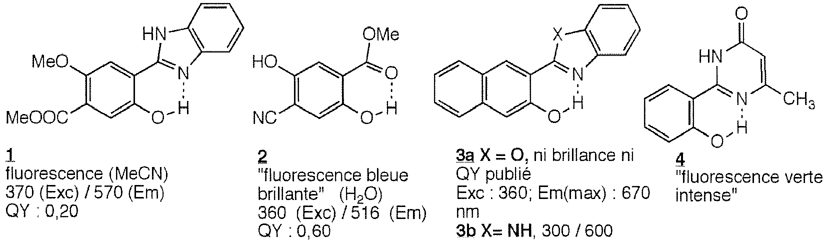 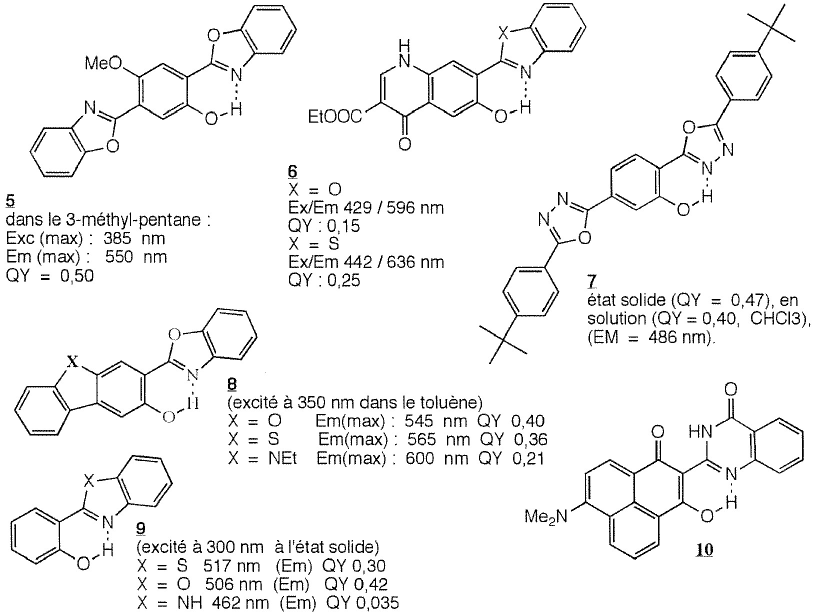
**1 :** Stefani, V., et al. Dyes and Pigments (1992)20, 97-107.
**2 :** Yamada, S., et al. Chemistry Letters (1999), 197-198.
**3a** : Nagaoka, S., et al. Journal of Photochemistry and Photobiology a-Chemistry (1999)122, 151-159.
**3b** : Douhal, A., et al. Chemical Physics Letters (1994)217, 619-625.
**4** : Kemp, D.S., et al., Journal of Organic Chemistry (1981)46, 1804-1807.
**5** :Mordzinski, A., et al. Journal of Physical Chemistry (1986)90, 1455-1458.
**6** : Lins, g.O.W., et al. Dyes and Pigments (2010)84, 114-120.
**7** :Seo, J., et al. Journal of Photochemistry and Photobiology a-Chemistry (2007)191, 51-58.
**8** : Kauffman, J. M., et al. Journal of HeterocyclicChemistry (1995)32, 1541-1555.
**9 :** Heller, A., et al. J. Phys. Chem. (1970)74, 4473-4480.
**10** : JP2004142131.

Ainsi, le groupe R₅ peut être choisi pour que le substrat de peptidase libère un fluorophore Ph-OH choisi parmi les fluorophores phénoliques ESIPT **1, 3a, 3b, 5, 6, 8, 9** et **10,** après clivage par la peptidase cible.

Tous les groupements peptidyle ou aminoacides possibles constituant des substrats de peptidases peuvent être utilisés dans R₀ présent dans les composés de formule (I), (IA) ou (IA'). Les aminoacides peuvent être fonctionnalisés ou non, en particulier sur l'extrémité N-terminale de R₀. Selon un mode de réalisation de l'invention, le résidu peptidyle a au plus 10 aminoacides qui peuvent être identiques ou différents. Selon un mode de réalisation particulier, pour des raisons de coût du substrat, le résidu peptidyle a au plus 6 aminoacides identiques ou différents. Les aminoacides du résidu peptidyle sont, de préférence, choisis parmi les aminoacides naturels. Néanmoins, l'extrémité N-terminale de l'aminoacide ou du groupe peptidyle peut être fonctionnalisée avec un groupe acyle -COR", R" étant un groupe (C₁-C₆)alkyle ou un groupe O-(C₁-C₆)alkyle. La fonctionnalisation possible de l'extrémité N-terminale d'une sonde donnée avec un groupe acyle (R"CO-) provient du fait que certaines endoprotéases n'interagiront pas avec un substrat ayant un groupe amino libre à son extrémité. Aussi, cette fonctionnalisation N-terminale sera préférée dans le cas où R₀ représente un groupe peptidyle, alors que dans le cas où R₀ représente un acide aminé, sa fonction N-terminale sera libre, ou de préférence salifiée sous la forme d'un ammonium. La synthèse peptidique en phase solide (SPPS) au moyen de groupes protecteurs de carbamate (qui constituent aussi des groupes "acyle" dans ce contexte) permet souvent de synthétiser plus simplement une sonde présentant un carbamate à cette extrémité N-terminale. Dans le cas des amino-peptidase, on préfèrera utiliser un groupe R₀ qui représente un acide aminé, alors que dans le cas des endopeptidases, on préfèrera utiliser un groupe R₀ qui représente un groupe peptidyle. Les modes de réalisation avec n=1 et R₁ qui représente une chaine latérale d'un amino acide tel qu'un groupe méthyle, iso-propyle, iso-butyle ou benzyle sont particulièrement avantageux dans les cas où R₀ un groupe peptidyle et non un amino-acide unique.

A titre d'exemples, les groupements peptidyle suivants peuvent être cités : Leu (pour la leucine aminopeptidase), Ser-Gln-Asn-Tyr (la partie N-terminale de la séquence de clivage préférée de la peptidase du VIH-1), Asp-Glu-Val-Asp- (pour la caspase 3), His-Ser-Ser-Lys-Leu-Gln (pour l'antigène spécifique à la prostate « PSA ») où l'extrémité N-terminale peut être libre ou être substituée par un groupe acyle -COR", R" étant un groupe (C₁-C₆)alkyle ou un groupe O-(C₁-C₆)alkyle (par exemple un groupe -COMe).

Le groupement peptidyle ou aminoacide est choisi pour son adéquation avec la sélectivité de séquence connue de la peptidase ciblée, qui le reconnaîtra. Le groupement peptidyle ou aminoacide peut être choisi pour l'action préférée qu'une peptidase impliquée dans certaines maladies va avoir sur ce dernier, comme présenté, notamment, dans le **Tableau 1.**

**Tableau 1 : Exemples de peptidases qui peuvent servir de biomarqueurs pour des phénomènes biologiques ou des maladies pendant l'imagerie**

| Peptidase | Classe | Fonction | Maladie |
|---|---|---|---|
| Leucine aminopeptidase | Zinc | Maturation post-protéasomale de peptides présentés de classe I | - |
| Caspase-3 | Cys | Apoptose | Cancer |
| Peptidase de VIH-1 | Asp | Réplication de VIH | SIDA |
| Rénine | Asp | Production d'angiotensine I | Hypertension |
| Thrombine | Ser | Coagulation sanguine | Infarctus du myocarde |
| Tryptase | Ser | Phagocytose | Asthme |
| Cathepsine K | Cys | Résorption osseuse | Ostéoporose |
| ACE | Zinc | Production d'angiotensine II | Hypertension |
| Plasmepsines I et II | Asp | Dégradation de l'hémoglobine | Malaria |
| β-Secrétase | Asp | Synthèse d'amyloïde β | Maladie d'Alzheimer |
| PSA (kallikrein III) | Ser | Liquification de l'éjaculat de sperme | Cancer de la prostate |

Les exemples **I.1** à **I.6** ciblent des membres de deux classes de peptidases différentes : une exopeptidase, plus spécifiquement une aminopeptidase (leucine aminopeptidase EC 3.4.11.1 pour **I.1** à **I.4**) et deux endopeptidases (a) caspase-3, EC 3.4.22.56 pour **I.5** ; b) PSA = « kallikrein III », EC 3.4.21.35 pour **I.6**). Tandis que les exopeptidases clivent seulement un résidu d'aminoacide de l'extrémité d'une chaîne peptidique, les endopeptidases sont, pour la plupart, des protéases vraies qui clivent une liaison peptidique à l'intérieur d'un squelette peptidique ; elles ont donc une spécificité de séquences plus ou moins élevée. La séquence de clivage de la caspase-3 préférée est : Asp-Glu-Val-Asp---Gly-Asp-. Pour la création de sondes fluorogènes, il suffit d'offrir à la peptidase seulement la partie N-terminale (à gauche de la liaison peptidique scissile) de la séquence préférée, c'est-à-dire Asp-Glu-Val-Asp- (ou dans le code à une lettre : DEVD), bien que ceci soit associé avec une perte par degrés du taux de transformation catalytique. Cependant, pour que la caspase reconnaisse DEVD, l'extrémité N-terminale doit être acétylée ; une extrémité amino-terminale libre annihilerait la reconnaissance par l'enzyme.

Les substrats (I) selon l'invention peuvent être obtenus par couplage d'une amine de formule (II) : 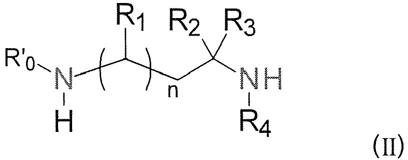 dans laquelle R₁, R₂, R₃ et R₄ sont tels que définis pour les substrats de formule (I) et R'₀ représente un groupe R₀ est tel que défini pour les substrats de formule (I) ou le plus souvent un tel groupe R₀ dans lequel la ou les fonctions amine ou acide présentes sont protégées
avec un dérivé chloré de formule (III) : 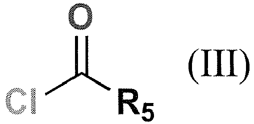 dans laquelle R₅ est tel que défini pour les substrats de formule (I), suivi éventuellement d'une déprotection des fonctions amine ou acide du substituant R'₀ pour donner R₀.

Un tel couplage peut notamment être réalisé en présence d'une base telle que la triéthylamine.

Les réactions de protection et déprotection sont effectuées selon des techniques bien connues de l'Homme de l'art. La protection des fonctions amine et acides sera effectuée par un groupe protecteur temporaire des amines, ou des acides carboxyliques bien connus de l'homme de l'art, tels que ceux décrits dans Protective groups in Organic Synthesis, Greene T.W. et Wuts P.G.M., ed John Wiley et Sons, 2006 et dans Protecting groups, Kocienski P.J., 1994, Georg Thieme Verlag.

A titre d'exemple de groupe protecteur des amines, et notamment de l'amine présente en bout de chaine de l'acide aminé ou du résidu peptidyle, on peut citer le groupe tert-butoxycarbonyle (Boc) et à titre d'exemple de groupe protecteur des fonctions acides, on peut citer le groupe tert-butyl qui conduit à la formation intermédiaire d'un ester.

Certaines synthèses de composés (II) sont décrites de manière détaillée dans les exemples qui vont suivre et l'homme du métier pourra procéder par analogie. Pour la préparation des composés (III), ces derniers pourront être obtenus à partir des phénols correspondants déjà décrits dans la littérature voire même commerciaux, par exemple, par action de triphosgène.

Les différents composés selon l'invention peuvent se trouver sous toutes les formes d'isomères optiques ou diastéréoisomères possibles, éventuellement en mélange selon toutes proportions, à moins qu'il n'en soit spécifié autrement. Selon un mode de réalisation particulier, les composés selon l'invention comportant un carbone asymétrique se trouvent sous une forme racémique, les formes R et S se trouvant en proportions sensiblement égales. Selon un autre mode de réalisation, les composés de formule (I) de l'invention peuvent se trouver sous une forme enrichie en un diastéréoisomère ou énantiomère, avec un excès diastéréoisomérique ou énantiomérique supérieur à 80 %, voire même supérieure à 95%, voire sous une forme isomérique pure c'est-à-dire avec un excès diastéréoisomérique ou énantiomérique supérieur à 99 %.

Les composés (I) sont isolés sous une forme enrichie en un diastéréoisomère ou énantiomère par les techniques classiques de séparation : on pourra utiliser, par exemple des recristallisations fractionnées d'un sel du racémique avec un acide ou une base optiquement active dont le principe est bien connu ou, le plus souvent, les techniques classiques de chromatographies sur phase chirale ou non chirale.

La présente invention rend l'activité des peptidases accessibles par imagerie par fluorescence utilisant des fluorophores ESIPT, par exemple, du type HPQ (ou d'analogue de HPQ). Aucun signal faussement positif dû à une dégradation spontanée (c'est-à-dire en l'absence de peptidase cible, en milieu physiologique) n'est observé. La sonde elle-même est peu ou n'est pas fluorescente (pas de fluorescence intrinsèque), en particulier à la longueur d'onde d'émission du fluorophore ESIPT libre sur laquelle l'instrument de détection/imagerie est réglé. La sonde fonctionne ainsi dans le mode éteint/allumé et peut être utilisée pour le développement d'analyses avec une sensibilité maximale. Cette invention permet de cibler des exo-(amino-)peptidases mais également des endopeptidases avec une sélectivité élevée pour des séquences d'aminoacides particulières. Ceci est possible par couplage d'une séquence peptidique spécifique d'une endoprotéase particulière à l'extrémité de l'espaceur intelligent.

De plus, la présente invention représente une amélioration par rapport à celle contenue dans Zhang, X.-B., Waibel, M., et Hasserodt, Chem. Eur. J.(2010), 16, 792-795, en ce que la présente sonde est caractérisée par une absence totale de dégradation spontanée dans les conditions physiologiques et ne conduit donc pas à la plus légère production d'un signal fluorescent faussement positif. Elle surmonte l'obstacle technologique de l'immolation lente des espaceurs diamines, si l'atome d'azote portant R₀ porte un hydrogène plutôt qu'un groupe alkyle qui est nécessaire pour cibler les peptidases qui, avec très peu d'exceptions (prolidases), clivent seulement les liaisons peptidiques secondaires et non pas les liaisons peptidiques tertiaires. La perte de vitesse de cyclisation qui en résulte (un facteur de dix, selon Saari, W.S., *et a*l., *supra*) est compensée par la préorganisation de l'espaceur diamine pour la formation de l'urée cyclique, le processus qui conduit à la libération du fluorophore ESIPT. L'énorme gain de vitesse de cyclisation qui est obtenu en préorganisant l'espaceur a déjà été démontré par la demanderesse pour deux systèmes moléculaires indépendants : Waibel, M., Zhang, X.-B., et Hasserodt, J. Synthesis (2008) 318-324 ; et Zhang, X.B., Waibel M., et Hasserodt, J. Chemistry-a European Journal (2010)16, 792-795. Dans le premier article, la fragmentation dans les conditions physiologiques était instantanée, dans le second, elle était très rapide. Cependant, aucun des deux systèmes ne bénéficie de la présence de la liaison carbamate tertiaire au phénol et présentait une dégradation résiduelle dans des conditions physiologiques.

Les substrats de la présente invention bénéficient d'une bonne perméabilité vis à vis de la membrane cellulaire par rapport à d'autres substrats d'enzymes fluorogènes connus et seront capables d'entrer aisément dans les cellules (à comparer avec : Duhamel, S. et al. Journal of Microbiological Methods 75 (2008) 269-278), de sorte que ces substrats pourront être utilisés pour différentes applications dans une grande variété de cellules. De plus, les substrats selon la présente invention sont généralement solubles mais très peu fluorescents sous une forme solubilisée dans l'eau (fluorescence intrinsèque), cependant, ils émettent un signal hautement fluorescent dans une solution aqueuse contenant le substrat et la peptidase correspondante. Dans le cas de la HPQ, ce signal fluorescent est émis à l'état solide par le précipité formé sous l'action de la peptidase car la HPQ est très insoluble dans la plupart des solvants, mais en particulier dans les milieux aqueux. D'autres fluorophores ESIPT ne sont pas non plus particulièrement solubles dans les milieux aqueux, mais la plupart conservent leur capacité à émettre une fluorescence même s'ils précipitent. Les conditions appropriées pour permettre la formation d'un précipité fluorescent lors de l'hydrolyse par une peptidase sont les milieux purement aqueux, comme un milieu tamponné ou un milieu physiologique. Un point important est que le précipité est formé lors de l'hydrolyse par une peptidase sans compromettre l'activité peptidase. Ainsi, leur utilisation pour des études de localisation dans des échantillons biologiques ou la détection de bandes discrètes en PAGE non dénaturante et en transfert de Western est possible. De plus amples détails concernant les conditions et les techniques de détection qui peuvent être utilisées sont donnés dans WO 2004/058787 et EP-0641351 qui peuvent être appliqués directement à la présente invention.

Les sondes selon l'invention sont attractives pour plusieurs applications à haute sensibilité dans les sciences de la vie, et notamment : (1) le criblage à haut rendement d'une activité peptidase exprimée par des colonies bactériennes sur une plaque gélosée (analyse sur colonies) ; (2)la détection *in vitro* de peptidases dans des liquides biologiques (hématologie et autres) ; (3) la visualisation d'une activité peptidase au niveau d'une simple cellule en cytométrie de flux; (4) la détection de peptidases subcellulaires dans des cellules cultivées (microscopie de fluorescence confocale) ; (5) la détection histochimique de peptidases (à l'échelle tissulaire) ; et finalement (6) l'imagerie *in vivo* d'un animal entier. Les sondes selon l'invention répondent aux exigences des experts dans le domaine de la création de sondes ayant une plus grande robustesse que celles qui sont disponibles actuellement (National Research Council of the U.S.A. (2006) Visualizing Chemistry : The Progress and Promise of Advanced Chemical Imaging, The National Academies Press). En particulier, le domaine à croissance rapide de l'imagerie moléculaire *in vivo* manque de sondes furtives intelligentes qui sont suffisamment robustes, et de complexité moléculaire limitée pour être fabriquées avec des coûts limités (Baker, M. (2010). Whole-animal imaging: The whole picture. Nature 463, 977-980).

Ainsi, les substrats de peptidase selon la présente invention ont un grand nombre d'applications potentielles. Les exemples de telles applications incluent :
(a) la conception d'analyses sur colonies bactériennes. Celles-ci sont actuellement réalisées sur une plaque gélosée (boîte de Pétri) où jusqu'à 3 000 colonies peuvent être distinguées sans avoir à les séparer activement dans des compartiments séparés comme les puits contenus dans une plaque à puits multiples. Il est ainsi possible de (1) concevoir des tests sur échantillons cliniques permettant d'identifier parmi un ensemble de lignées bactériennes une lignée pathogène d'intérêt ; (2) réaliser des tests parallèles massifs d'une banque de protéines de sa propre production exprimée par un hôte bactérien classique (souvent commercial). Cette collection de protéines peut bien entendu contenir une protéine d'intérêt particulier, par exemple une protéase ayant une sélectivité pour une séquence d'aminoacides spécifique, ou une protéase hydrolysant une liaison carboxamide non naturelle. Dans le domaine de l'évolution dirigée des protéines en général ou des enzymes en particulier, il existe une forte demande pour des analyses efficaces et sensibles pour cribler de très grands nombres de variants de protéines dépassant aisément 10⁶. L'application de la sonde selon l'invention peut être envisagée le plus aisément par dissolution dans la solution gélosée avant qu'elle soit versée dans la plaque où elle se gélifie. A titre d'alternative, les substrats sont incubés avec les colonies par immersion d'un filtre avant qu'il soit pressé sur les colonies. Le principal avantage auquel la sonde selon l'invention contribue pour une telle analyse sur colonies est la précipitation sur site du fluorophore ; une dilution du signal fluorescent par diffusion est donc très réduite, ce qui permet de plus grandes durées d'incubation et donc une plus grande sensibilité pour l'analyse. Le très grand déplacement de Stokes de la HPQ (approximativement 140 nm) ou de tout analogue de la HPQ ne devrait pas rester mésestimé ; il contribue aussi à l'excellente sensibilité et la rend aisément distinguable de la fluorescence native provenant de l'échantillon biologique.
(b) L'imagerie in vitro (histologie) et in vivo. Compte tenu de la très faible solubilité de la HPQ libre ou de tout analogue de la HPQ, toute libération de celle-ci dans un environnement biologique complexe permet l'imagerie par fluorescence avec un haut contrôle spatial. L'imagerie par fluorescence est une technique largement appliquée pour mettre en lumière les structures subcellulaires. La localisation d'une activité peptidase spécifique à haute résolution peut être possible par l'utilisation d'une sonde selon l'invention.

Au contraire, les sondes fluorescentes passives (celles qui ne sont pas transformées par une enzyme) sont équipées d'un ligand spécifique d'un récepteur cellulaire pour se fixer sur ce récepteur par des interactions non covalentes ; elles ne sont donc pas dépourvues d'effets de dilution du signal. Par-dessus tout, un récepteur équivaut au mieux à un marqueur fluorescent. La sensibilité d'une analyse basée sur de telles sondes est nécessairement bien plus basse que celle d'une sonde active qui bénéficie de l'amplification catalytique du signal.

Les sondes selon l'invention peuvent servir aussi pour l'imagerie par fluorescence macroscopique, c'est-à-dire au niveau d'un organisme entier. Si la sonde pénètre dans la paroi cellulaire pour atteindre l'activité peptidase d'intérêt, la libération du fluorophore libre exclue la dilution rapide du signal par partage avec l'espace extracellulaire. Généralement, ceci permet des durées d'incubation prolongées avant l'imagerie, qui sont particulièrement utiles quand l'activité enzymatique est exprimée faiblement.

Les substrats de peptidase selon l'invention peuvent être préparés selon des techniques connues, détaillées dans les exemples suivants. Par exemple, un groupement peptidyle ou aminoacide éventuellement protégé peut être greffé sur le groupe espaceur. Après une étape de couplage pour introduire le fluorophore *via* son hydroxyle phénolique, le groupement peptidyle ou aminoacide peut être déprotégé. La sonde complète ainsi obtenue peut être purifiée, avant l'utilisation, par des techniques conventionnelles.

Les exemples suivants illustrent l'invention, mais n'ont aucun caractère limitatif.

### Généralités

La chromatographie sur colonne a été réalisée sur gel de silice 60-mesh (40-63 µm). Les spectres de RMN de ¹H et de ¹³C ont été enregistrés à 200,13 MHz et à 50,13 MHz, respectivement, dans le chloroforme deutéré, sauf indication contraire. Les déplacements chimiques (δ) sont indiqués en ppm et notés en référence au tétraméthylsilane ou d'après des signaux résiduels du solvant ; les abréviations s = singulet, d = doublet, t = triplet, m = multiplet, br = large sont utilisées. Les constantes de couplage de RMN (J) sont indiquées en Hertz. Les analyses par fluorescence ont été réalisées dans des plaques en polypropylène noir à 96 (Corning, Corning Inc.) ou à 384 (NUNCLONE, Nunc Inc.) puits, et enregistrées sur un fluorimètre à microplaques (Mithras LB940 de Berthold Technologies). Sauf quand cela est spécifié, les produits chimiques ont été achetés avec la qualité de réactif analytique et utilisés sans autre purification.

Le THF et le DCM secs commerciaux ont été séchés et purifiés par passage sur colonne d'alumine activée sous argon (GT S100 Solvant Station System). La triéthylamine a été distillée à partir de l'hydrure de calcium et conservée sur des pastilles de KOH. Les autres réactifs notés secs ont été séchés sur tamis moléculaires. Si le contraire n'est pas indiqué, toutes les réactions ont été conduites sous atmosphère d'air avec des solvants et réactifs commerciaux, sans séchage ou purification supplémentaire. De l'eau millipore obtenue à partir d'un système de purification Elga Purelab a été utilisée dans toutes les expériences.
Les abréviations suivantes sont utilisées :
Me = méthyle
Et = éthyl
cbz = carbobenzoxy
tBu = Butyle tertiaire
Boc = tert-butoxycarbonyl
DCC = dicyclohexylcarbodiimide
TEA = triéthylamine
pTsOH = acide para-toluène-sulfonique
EA = acétate d'éthyle
Cy = cyclohexane
HOBt = N-hydroxybenzotriazole
TFA = acide trifluoroacétique
THF = tetrahydrofuran
Leu = L-Leucyl
BocLeu = N-(tert-butoxycarbonyl)-L-leucyl
EDC = Chlorhydrate de 1-éthyl-3-(3,5-diméthylaminopropyl)-carbodiimide
CCM = chromatographie sur couche mince
DMF = diméthylformamide
DCM = dichlorométhane
DDQ = 2,3-dichloro-5,6-dicyano-1,4-benzoquinone
DCU = di-cyclohexylurée
PLA = leucine aminopeptidase microsomale

### Exemple I.1

Le substrat **I.1** est préparé comme décrit sur le **Schéma 5** suivant.

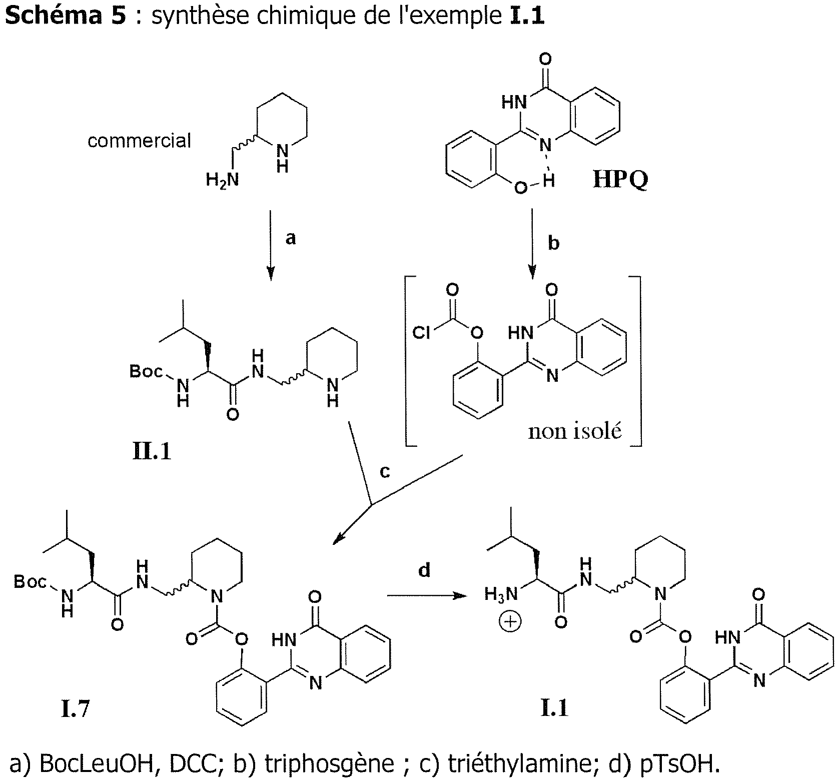

**Préparation du composé II.1** : BocLeuOH (4,385 g, 19,0 mmol ; commerce ; Fluka, 15450, ≥ 99.0%) est totalement dissout dans du DCM sec (50 mL) sous une atmosphère anhydre, avant de porter la température du mélange à 0°C avec un bain de glace. Du DCC (3,95 g, 19,1 mmol) est rajouté et le mélange agité pendant 5 minutes avant l'addition de l'aminométhylpipéridine (2,11 g, 18,5 mmol ; commerce ; Aldrich 656518, 97%). Le mélange est porté à température ambiante en retirant le bain de glace. Après 24 heures d'agitation, la DCU précipitée sous forme d'un solide blanc est éliminée par filtration sur fritté de porosité 3 et le solide rincé avec du DCM. La solution jaune obtenue est lavée avec du Na₂CO₃ saturé, puis avec du tampon phosphate à 0,5 M et à pH >10, lavée ensuite par de la saumure, séchée sur Na₂SO₄ qui est éliminé par filtration, et évaporée, afin de donner une huile jaune (6,6 g). Cette huile est conservée à 4°C pendant une nuit, ce qui fait précipiter du DCU qui est éliminé avec la même procédure décrite ci-dessus. Le spectre RMN du brut indique la présence du produit souhaité presque pur, très légèrement contaminé par un produit parasitaire habituel des couplages DCC, à savoir la DCU acylé par l'acide aminé. Une purification sur colonne de silice avec un gradient EA:Cy:MeOH (1:1:0=>1:1:1, v :v :v) donne le produit **II.1** pur sous forme d'un solide incolore (2,703 g ; 42 %). Les spectres RMN (a) montrent la présence de deux diastéréoisomères dans un rapport molaire 1:1 due à la présence du centre stéréogène au niveau du cycle pipéridine et (b) semblent être compliqués par la présence de différents conformères du cycle.
¹H-RMN (200 MHz, CDCl₃): 7,09-6,81 ((s, br + s, br), 1H), 5,70-5,34 ((s, br + d, br, 7,6 Hz), 1H), 4,08-3,98 (m, 1H), 3,31-3,15 (m, 1H), 3,11-2,90 (m, 2H), 2,70-2,44 (m, 2H), 2,14 (s, br, 1H, déplacement sensible au solvant et à la concentration, NH), 1,76-1,41 (m, 6H), 1,33 (s, 9H), 1,32-1,00 (m, 3H), 0,88 (d, 2.1Hz, 3H), 0.87 (d, 2.4Hz, 3H) ppm.
¹³C-RMN (125 MHz, CDCl₃): Diastereoisomère 1 : 173,0; 155,7; 79,6; 55,8; 53,2; 46,5; 45,0; 41,3; 30,1; 28,3 (x3); 26,3; 24,7; 24,2; 22,9; 22,0 ; Diastereoisomère 2 : 173,1; 155,8; 79,7; 55,5; 53,2; 46,5; 45,1; 41,2; 30,2; 28,3 (x3); 26,2; 24,7; 24,2; 22,9; 22,0 ppm. Des spectres HSQC, COSY et jmod rendent l'attribution des signaux complète.
HRMS (TOF MS ESI+) calculé pour [C₁₇H₃₄N₃O₃]⁺ = [MH]⁺: m/z = 328.2595, trouvé 328.2589.

**Préparation du composé I.7 :** Dans un bi-col contenant du HPQ (76 mg ; 0,32 mmol) sous une atmosphère d'argon anhydre est ajouté de la TEA sèche (57 mg ; 0,56 mmol ; 1,8 eq) et du DCM sec (1,2 mL) et le mélange est refroidi à 0°C. Sous agitation, une solution de triphosgène (124 mg ; 0,42 mmol) dans du DCM sec (1,2 mL) est rapidement injectée. Le mélange est agité à 0°C pendant 40 minutes, puis pendant 20 minutes à 25°C, avant d'être évaporé à sec à l'aide d'une pompe à palettes protégée par deux pièges à l'azote liquide. Le solide beige résultant est traité à température ambiante avec une solution de composé **II.1** (19 mg ; 0,058 mmol ; 0,2 eq) dans de la TEA sèche (101 mg ; 1 mmol ; 3 eq) et du DCM sec (1,2 mL). La solution de couleur vert olive est agitée pendant deux heures avant d'être versée sur du NaHCO₃ saturé. La phase aqueuse est extraite avec du CHCl₃, et les phases organiques sont rassemblées et lavées avec du NaHCO₃ saturé et de la saumure, puis séchées avec du Na₂SO₄ qui est éliminé par filtration, et évaporées à sec. Le résidu est repris dans du CHCl₃ et filtré deux fois sur Célite afin d'éliminer une partie de l'HPQ restant, et le filtrat est évaporé pour donner un solide pâteux verdâtre (70 mg). Une purification sur colonne de silice (gradient EA:Cy:MeOH, 2:2:0=>2:2:1, v :v :v) conduit au composé **I.7** pur (15 mg ; 0,025 mmol ; 44 %). Les spectres RMN (a) montrent la présence de deux diastéréoisomères dans un rapport molaire 1:1 due à la présence du centre stéréogène au niveau du cycle pipéridine et (b) semblent être compliqués par la présence de différents conformères du cycle, ce qui donne jusqu'à quatre résonances par noyau unique.
¹H-RMN (500 MHz, CDCl₃) : δ = 8,60 (s, br ; 0,5H) ; 8,36-7,92 (m, 2H) ; 7,89-7,78 (m ; 2,5H) ; 7,58-7,47 (m ; 2H) ; 7,39-7,35 (m ; 1H) ; 7,31-7,15 (m ; 1H) ; 6,86-6,81 (m ; 0,1H) ; 5,66-5,60 (m ; 0,1H) ; 5,05-4,88 (d, br + d,br ; 8,5 & 6.5 Hz ; 1H ; NH_{carbamate}) ; 4,71-4,66 (m ; 0,8H) ; 4,46-4,34 (m ; 1H) ; 4,20-3,94 (m ; 1H) ; 3,87-3,51 (m ; 1H) ; 3,43-2,89 (m ; 2H) ; 1,77-1,43 (m ; 9H) ; 1,09-0,84 (m, 15H) ppm.
¹³C-RMN (125 MHz, CDCl₃): δ = 174,6 ; 174,0 ; 171,3 ; 163,4 ; 162,9 ; 156,1 ; 155,7 ; 153,5 ; 152,8 ; 151,5 ; 151,2 ; 149,9 ; 149,7 ; 149,6 ; 149,4 ; 134,9 ; 134,9 ; 134,8 ; 132,1 ; 131,9 ; 130,7 ; 129,8 ; 128,3 ; 128,0 ; 127,1 ; 126,8 ; 126,6 ; 126,3 ; 126,1 ; 124,6 ; 123,1 ; 121,1 ; 120,9 ; 79,8 ; 54,4 ; 53,1 ; 52,6 ; 50,8 ; 43,6 ; 41,6 ; 41,3 ; 41,2 ; 39,9 ; 39,8 ; 39,7 ; 38,8 ; 29,4 ; 28,3 ; 28,2 ; 28,0 ; 27,1 ; 26,9 ; 25,6 ; 25,3 ; 24,9 ; 23,0 ; 22,3 ; 19,6 ; 19,3 ppm.
HRMS (TOF MS ESI⁺) calculé pour [C₃₂H₄₂N₅O₆]+= [MH⁺]: m/z 592,3130 ; trouvé 592,3123.

**Préparation du composé I.1 :** Le composé **I.7** (174 mg ; 0,294 mmol) est dissout dans de l'éthanol sec (10 mL) contenant du pTsOH.H₂O (61 mg, 0,32 mmol), mis sous une atmosphère d'argon et chauffé à 80°C pour 8 heures. La réaction est suivie par CCM (EA:Cy:MeOH 1:1:1, v:v:v ; UV et ninhydrine). Des traces d'HPQ peuvent apparaître. La solution brute est soumise à une purification sur colonne de silice et sont successivement obtenues des traces d'HPQ (EA:Cy:MeOH 1:1:0, v:v:v), des traces résiduelles de **I.7** (EA:Cy:MeOH 1:1:0,15, v:v:v), et le produit **I.1** brut (EA:Cy:MeOH 1:1:0,3, v:v:v) sous la forme d'un solide (149 mg) qui fluoresce légèrement bleu sous une lampe UV. Sa trituration avec de l'eau enlève un éventuel excès de TsOH et le solide restant (107 mg) donne un spectre ¹H-RMN qui indique un rapport molaire 1:1 **I.1**:tosylate. Ce solide est peu soluble dans l'eau mais peut être facilement dissout dans du MeOH (0,6 mL) avant d'être dilué avec de l'eau sans crainte de précipitation. Les spectres RMN (a) montre la présence de deux diastéréoisomères dans un rapport molaire 1:1 due à la présence du centre stéréogène au niveau du cycle pipéridine et (b) semblent être compliqués par la présence de différents conformères du cycle, ce qui donne jusqu'à quatre résonances par noyau unique.
¹H-RMN (D₂O, 500 MHz) : δ = 8,20 (d ; 8,1 Hz ; 1H) ; 7,94 (t apparent ; 7,9 Hz ; 1H) ; 7,77-7,70 (m ; 3H) ; 7,68-7,65 (m ; 1H) ; 7,51-7,47 (m ; 1H) ; 7,36-7,32 (m ; 1H) ; 4,52-2,77 (m ; 6H) ; 1,65-1,22 (m ; 9H) ; 0,81-0,70 (m ; 6H) ppm.
¹³C-NMR (D₂O, 125 MHz) : δ = 170,5 ; 170,3 ; 162,3 ; 162,2 ; 154,1 ; 153,9 ; 153,8 ; 148,2 ; 148,1 ; 141,7 ; 141,3 ; 137,1 ; 137,1 ; 135,1 ; 134,8 ; 130,2 ; 130,2 ; 129,6 ; 127,0 ; 123,6 ; 123,4 ; 123,3 ; 122,4 ; 122,3 ; 122,1 ; 122,0 ; 119,2 ; 52,0 ; 51,9 ; 51,6 ; 51,4 ; 50,9 ; 40,5 ; 40,0 ; 40,0 ; 39,9 ; 38,2 ; 25,8 ; 25,0 ; 25,0 ; 24,5 ; 23,9 ; 23,9 ; 21,8 ; 20,9 ; 20,8 ; 18,2 ; 18,0 ppm.
MS (ESI, mode positif): m/z 492,3 : [MH]⁺. MS (ESI, mode négatif): m/z 490,2 : [M-H]⁻.
L'analyse par HRMS (TOF MS ESI⁺) sur le cation a été exécutée sur un échantillon présentant les même spectres RMN mais préparé en appliquant sensiblement le même protocole utilisé pour la préparation du composé **I.2** (le composé **I.7** est alors utilisé à la place du composé **I.8**); dont calculé pour [C₂₇H₃₄N₅O₄]⁺ = [MH⁺]: m/z 492.2605, trouvé 492.2592.

### Exemple 1.2

Le substrat **I.2** est synthétisé comme décrit ci-dessus pour le substrat **I.1** sauf qu'à la place de l'aminométhyl-pipéridine (commercial), il faut tout d'abord synthétiser la N²-2-diméthylpropane-1,2-diamine **(13)** en trois étapes selon des protocoles adaptés de la littérature.

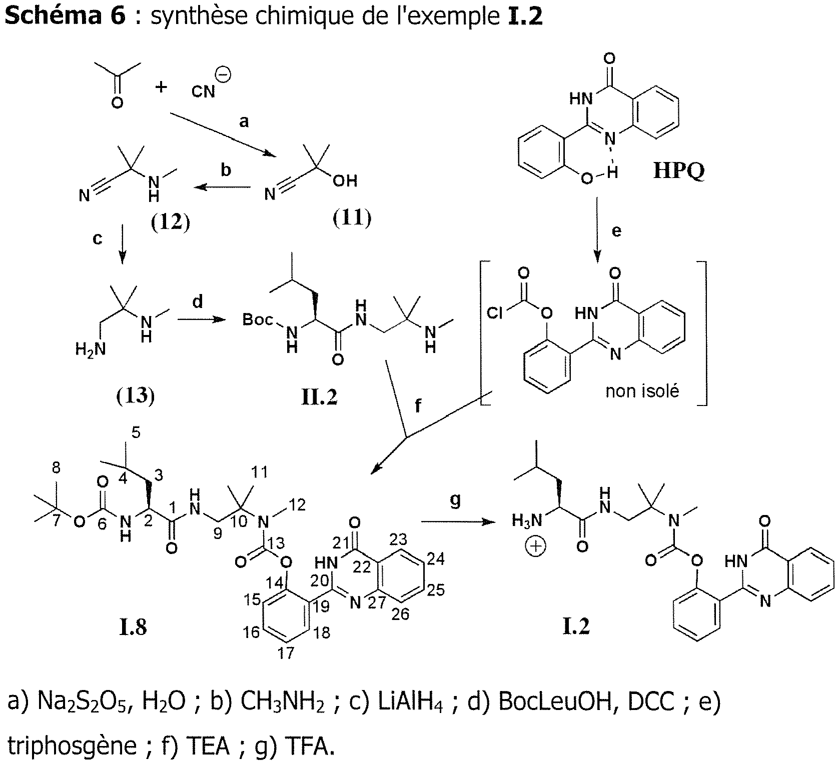

**Préparation du composé (11)** : (acétone cyanohydrine ; adapté de Faghihi, K.; Zamani, K.; Mirsamie, A.; RezaSangi, M., Eur. Polym. J. 2003, 39 (2), 247-254.) Une solution aqueuse (20 mL) de metabisulfite de sodium (11 g, 58 mmol) doucement agitée est traitée avec de l'acétone (5,8 g, 100 mmol). L'addition d'une solution aqueuse (20 mL) de cyanure de potassium (6 g, 92 mmol) entraine la séparation de l'acétone cyanohydrine désirée dans une seconde phase (phase du dessus). Une fois cette réaction terminée, les deux phases sont séparées et celle du dessus est séchée sur Na₂SO₄ et filtrée pour donner l'acétone cyanohydrine **(11)** pure sous la forme d'un liquide clair (5,402 g, 69 %). ¹H-NMR (200 MHz, CDCl₃): δ = 1,43 (s, 6H, 2×CH₃) ppm; ¹³C-NMR (50 MHz, CDCl₃): δ = 29,4 (2×CH₃), 65,3 (C(Me)₂), 123,0 (C≡N) ppm ; MS (ESI, mode positif): m/z 86,1 : [MH]⁺.

**Préparation du composé (12)** : (2-méthylamino-2-méthyl-propionitrile ; adapté de Exner, L. J.; Luskin, L. S.; de Benneville, P. L., J. Am. Chem. Soc. 1953, 75(19), 4841-4842.) Une solution aqueuse (40 % en masse) de méthylamine (2,8 g, 36 mmol) est ajoutée lentement à l'acétone cyanohydrine **(11)** (1 g, 12 mmol) située dans un ballon placé dans un bain de glace. L'addition est effectuée lentement, afin que la température du mélange n'excède pas 15 °C. Une fois l'addition terminée, le mélange réactionnel est agité pendant une heure et demie. La solution obtenue est extraite avec Et₂O (3×10 mL). Les phases organiques sont combinées et séchées sur Na₂SO₄ qui est ensuite éliminé par filtration. Le solvant est éliminé sous pression réduite pour conduire au produit **(12)** sous la forme d'un liquide incolore (0,700 g, 59 %). ¹H-RMN (200 MHz, CDCl₃): δ = 1,21 (s, 6H, 2×CH₃), 2,27 (s, 3H, NCH₃) ppm; ¹³C-RMN (50 MHz, CDCl₃): δ = 27,5 (2×CH₃), 31,8 (NCH₃), 52,5 (C(Me)₂), 122,8 (C≡N) ppm ; MS (ESI, mode positif): m/z 99,1 [MH]⁺.

**Préparation du composé (13)** : (N²-2-diméthylpropane-1,2-diamine; adapté de US 2005/0038078). Le 2-méthylamino-2-méthyl-propionitrile **(12)** (1,960 g, 20 mmol) est additionné goutte à goutte à une suspension d'hydrure de lithium et d'aluminium (1,520 g, 40 mmol) dans Et₂O (2 mL) sous atmosphère d'argon. Le mélange est agité pendant 3 heures à température ambiante, puis est dilué dans Et₂O (30 mL). Une fois placé dans un bain de glace, une solution aqueuse saturée de K₂CO₃ est ajouté jusqu'à ce que plus aucune émission d'hydrogène ne soit observée. Du Na₂SO₄ anhydre est alors ajouté au mélange réactionnel qui est agité pendant 10 min. L'élimination des sels est effectuée par filtration sur Célite qui est ensuite lavé avec des grands volumes d'Et₂O. Le filtrat obtenu est alors concentré sous pression réduite pour donner la N²-2-diméthylpropane-1,2-diamine **(13)** sous la forme d'un liquide clair (2,001 g, 98%). ¹H-RMN (200 MHz, CDCl₃): δ = 2,40 (s, 2H), 2,14 (s, ∼3H, NHMe), 1,8 (s, br, 3H, NH + NH₂), 0,87 (s, 6H, 2×Me) ppm ; ¹³C-RMN (50 MHz, CDCl₃): δ = 53,0 (CMe₂), 50,2 (CH₂), 28,3 (NHMe), 23,0 (2×Me) ppm ; MS (ESI, mode positif) : m/z 103,1 : [MH]⁺.

**Préparation du composé II.2** : Une solution de BocLeuOH (1,37 g ; 5,92 mmol) dans du DCM sec (50 mL) à - 5°C est traitée avec du DCC (1,227 g ; 1 eq) et agitée pendant 5 minutes. N²-2-diméthylpropane-1,2-diamine **(13)** (1,50 g d'une solution à 47 % en masse dans du Et₂O ; > 1 eq) est rajoutée avant de porter la température du mélange à l'ambiante et laisser le mélange agiter pendant 48 heures. Le DCU précipité (incolore) est filtré sur un fritté en verre de porosité 3. Le filtrat presque incolore est lavé avec du Na₂CO₃ saturé, afin d'éliminer le BocLeuOH résiduel. La solution en DCM est ensuite lavée avec du HCl (2M, 3x30 mL) afin d'éliminer toutes les composants amines. La solution DCM résultant ne contient alors que le sous-produit urée acylé par l'acide aminé. La solution aqueuse acide est tout de suite basifiée avec du KOH (5M, 40 mL), afin d'ajuster le pH à >12, et ensuite lavée avec du DCM (6×20 mL) jusqu'à ce que les deux phases deviennent limpides. Les phases organiques recombinées sont lavées avec de la saumure et séchées sur du Na₂SO₄. Après filtration et évaporation, on obtient 0,54 g d'une huile limpide comprenant le composé **II.2** (43 mol%, 52 wt%, 280 mg ; 0,88 mmol) et son dérivé qui a perdu le groupement Boc (H₂NLeu-NH-CH₂-C(Me)₂-NHMe, MW = 215.3 Da, 57 mol%, 48 wt%, 260 mg,1,2 mmol). Ce mélange est filtré sur une colonne de silice en utilisant un gradient de EA:Cy:MeOH (10:7:0=>10:7:3, v:v:v), afin d'obtenir uniquement le produit **II.2** sous la forme d'une huile incolore qui se solidifie au cours du temps (235 mg ; 0,74 mmol, 13%). Contrairement aux spectres RMN du composé **II.1,** ceux du composé **II.2** confirment l'absence attendue de diastéroisomères. Les chiffres numérotant les résonances RMN font référence à la numérotation indiquée sur le **Schéma 6** de synthèse pour les atomes correspondants du composé **I.8.**
¹H NMR (500 MHz, CDCl₃): δ = 6,80 (s, br ; 1H ; NH_{amide}) ; 5,04 (∼d, br ; 7,7 Hz ; 0,8H ; NH_{Boc}) ; 4,12-4,08 (m ; 1H ; H-2) ; 3,26 (dd ; 14,0 & 5,9 Hz ; 1H ; H-9) ; 3,17 (dd ; 13,3 & 4,9 Hz ; 1H ; H-9') ; 2,34 (s ; 3H ; 3×H-12) ; 2,14 (s, br ; 1H ; NH_{aliphatique}) ; 1,72-1,65 (m ; 2H ; H-4 + H-3) ; 1,54-1,49 (m ; 1H ; H-3') ; 1,46 (s ; 9H ; 9×H-8) ; 1,11 (s ; 6H ; 6×H-11) ; 0,96 (d ; 3,7Hz ; 3H ; 3×H-5) ;0,95 (d ; 3,6 Hz ; 3H ; 3xH-5') ppm.
¹³C NMR (125 MHz, CDCl₃): δ = 172,9 (C1) ; 155,8 (C6) ; 80,0 (C7) ; 53,7 (C10) ; 53,5 (C2) ; 46,6 (C9) ; 41,4 (C3) ; 28,4 (3×C8) ; 28,3 (C12) ; 24,9 (C4) ; 24,6 (2×C11) ; 23,0 (C5) ; 22,2 (C5') ppm. Des spectres HSQC, COSY et jmod ont été mesurés afin de confirmer l'attribution des signaux.
MS (ESI, mode positif) : m/z 316,3 : [MH]⁺.

**Préparation du composé I.8** : En appliquant sensiblement le même protocole utilisé pour la préparation du composé **I.7,** l'HPQ (149 mg ; 0,628 mmol) et le composé **II.2** (198 mg ; 0,627 mmol) sont introduits, afin de préparer le composé **I.8.** Le brut de la réaction est traité avec de la pipérazine dans de la pyridine sec, afin de piéger tout chloroformiate de HPQ qui n'aurait pas réagi avec l'amine. Le traitement aqueux décrit précédemment fournit une huile jaune brute (299 mg) qui contient le composé **I.8,** de l'HPQ, et des traces du produit résultant de la réaction avec la pipérazine. Une partie du brut (200 mg) est purifié dans la manière suivante. Le solide est traité avec du DCM (2×1 mL) et la partie soluble séparée par décantation. Celle-ci est ensuite purifiée par chromatographie sur colonne de silice (gradient Cy:EA 3:1=>1:1, v:v) afin de donner le composé **I.8** sous forme d'un poudre dense, incolore, et très légèrement bleu fluorescent (134 mg ; 0,231 mmol ; rdt. total est alors de 55%).
¹H-RMN (500 MHz, CDCl₃): δ = 8,29 (d ; 7,8 Hz ; 1H ; H-23) ; 7,91 (d ; 7,3 Hz ; 0,8H ; H-18) ; 7,80-7.78 (m ; 2,2H ; H-25 + H-16) ; 7,55-7,48 (m ; 2H ; H-24 + H-26) ; 7,39-7,35 (m ; 1H ; H-17) ; 7,24 (d ; 8,3 Hz ; 1H ; H-15) ; 6,84 (s, br ; 0,8H ; H_{amide}) ; 6,18 (s, br ; 0,1H ; H_{amide}) ; 5,60 (d ;br ; 6,8 Hz ; 0,7H ; NH_{carbamate}) ; 4,23-4,03 (m ; 1H ; H-2) ; 3,81-3,37 (m ; 2H ; 2×H-9) ; 3,05 (s ; 3H ; 3×H-12) ; 1,80-1,49 (m ; 3H ; H-4 + 2×H-3) ; 1,34 (s ; 6H ; 6×H-11) ; 1,23 (s ; 9H ; 9×H-8) ; 0,95 (d ; 6,6 Hz ; 3H ; 3×H-5) ; 0,93 (d ; 6,5 Hz ; 3H ; 3×H-5') ppm.
¹³C-RMN (125 MHz, CDCl₃): δ = 173,8 (C1) ; 162,5 (C21) ; 155,8 (C6) ; 154,8 (C13) ; 150,7 (C20) ; 149,4 (C27) ; 149,2 (C14) ; 134,9 (C25) ; 132,3 (C16) ; 130,7 (C18) ; 127,9 (C24) ; 127,6 (C23) ; 127,2 (C26) ; 126,6 (C22) ; 126,4 (C17) ; 123,8 (C15) ; 121,1 (C19) ; 79,5 (C7) ; 60,9 (C10) ; 53,6 (C2) ; 46,5 (C9) ; 41,2 (C3) ; 32,7 (C12) ; 28,3 (3×C8) ; 25,3 (C4) ; 25,0 (C11) ; 24,9 (C11') ; 23,2 (C5) ; 22,0 (C5') ppm.

Des spectres HSQC et COSY ont été mesurés afin de confirmer l'attribution des signaux. Les chiffres numérotant les résonances RMN font référence à la numérotation indiquée sur le **Schéma 6** de synthèse.
MS (ESI, mode positif) : m/z 580.3 : [MH]⁺. MS (ESI, mode négatif): m/z 578.3: [M-H]⁻.

**Préparation du composé I.2 :** 56 mg de **I.8** (0,097 mmol) sont traités avec du DCM sec (1,5 mL) et du TFA (1,5 mL) et la solution résultante est agitée à température ambiante pendant une heure avant que toutes les composantes volatiles soient éliminées sous pression réduite. Une analyse CCM du brut (Cy:EA:MeOH 1:1:1 ; révélation par analyse UV, ninhydrine et KMnO₄) prouve la consommation totale du composé **I.8** et l'absence d'HPQ. Néanmoins, une chromatographie sur colonne de silice (gradient Cy:EA:MeOH 1:1:0=>1:1:1, v:v:v) est menée pour donner le composé **I.2** (poudre dense et incolore ; 48 mg ; 0,081 mmol ; 84%). Les chiffres numérotant les résonances RMN font référence à la numérotation indiquée sur le **Schéma 6** de synthèse.
MS (ESI, mode positif): m/z 480.2 :[MH]⁺. MS (ESI, mode négatif): m/z 478.3: [M-H]⁻.
¹H-RMN (500 MHz ; D₂O): δ = 8,02 (d ; 8,2 Hz ; 1H ; H-23) ; 7,81-7,77 (m ; 1H ; H-25) ; 7,64 (dd ; 7,4 & 1,7 Hz ; H-18) ; 7,60-7,56 (m ; 2H ; H-16 + H-26) ; 7,50-7,47 (m ; 1H ; H-24) ; 7,40-7,37 (m ; 1H ; H-17) ; 7,24 (d ; 8,2 Hz ; 1H ; H-15) ; 3,81 (t ; 7,4 Hz ; 1H ; H-2) ; 3,41 (d, br ; 13,4 Hz ; 1H ; H-9) ; 3,08-3,00 (m ; 1H ; H-9') ; 2,93 (s ; 3H ; 3×H-12) ; 1,54-1,41 (m ; 3H ; H-4 + 2×H-3) ; 1,02-0,83 (m ; 6H ; 6×H-11) ; 0,81 (d, 4,9Hz + d, 4,9Hz ; 3H + 3H ; 6×H-5) ppm.
¹³C NMR (125 MHz ; D₂O): δ = 170,4 (C1) ; 164,4 (*C21) ; 154,5 (*C13) ; 151,6 (*C27) ; 148,3 (C14) ; 147,8 (*C20) ; 136,0 (C25) ; 132,9 (C16) ; 129,7 (C18); 128,1 (C24) ; 126,5 (C23) ; 126,2 (C26) ; 126,0 (*C22) ; 126,0 (C17); 123,3 (C15) ; 119,7 (C19) ; 59,4 (C10) ; 52,0 (C2) ; 45,9 (C9) ; 40,0 (C3) ; 32.1 (C12) ; 24.0 (C4), 23.8 (2×C11), 21.6 (C5), 21.2 (C5') ppm. Des spectres HSQC, HMBC, COSY et udeft (ce dernier pour les cinq carbones quaternaires étoilés qui relaxent très lentement et ont alors très peu de signal ; voir Piotto et al, Magn. Reson. Chem. 2006 (44), 943-947) ont été mesurés afin de confirmer l'attribution des signaux.
MS (ESI, mode positif): m/z 480.2 :[MH]⁺. MS (ESI, mode négatif): m/z 478.3: [M-H]⁻.

### Exemple I. 3

Le substrat **I.3** est synthétisé comme décrit ci-dessus pour le substrat **I.1** en remplaçant l'intermédiaire synthétique **II.1** par le composé **II.3** dans le **Schéma 7** ci-dessous. La diamine protégée par un groupement protecteur cbz **(14)** est préparée par réduction de proline-amide protégée par cbz du commerce avec le borane BH₃, dans un reflux de THF avec un rendement publié de 74 % (Wang, J., et al. Chem. Eur. J. (2006)12, 4321-4332).

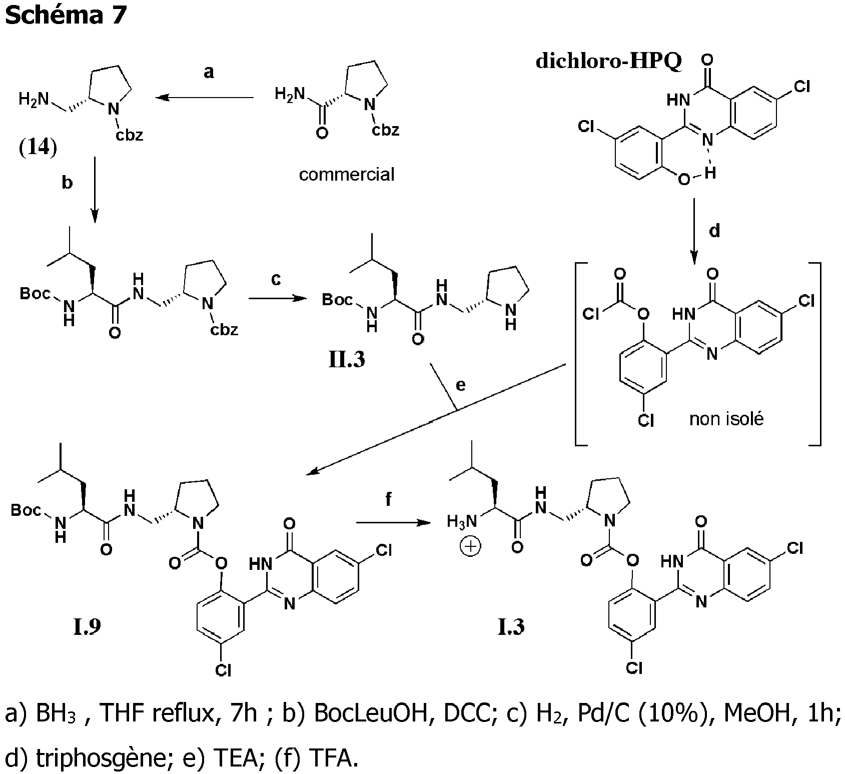

### Exemple I. 4

La sonde **I.4** est synthétisée de manière analogue à la sonde **I.1** avec remplacement de l'HPQ par le produit **6.**

### Exemple I.10

La sonde **I.5** est préparée comme décrit sur le **Schéma 8** suivant.

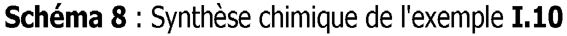

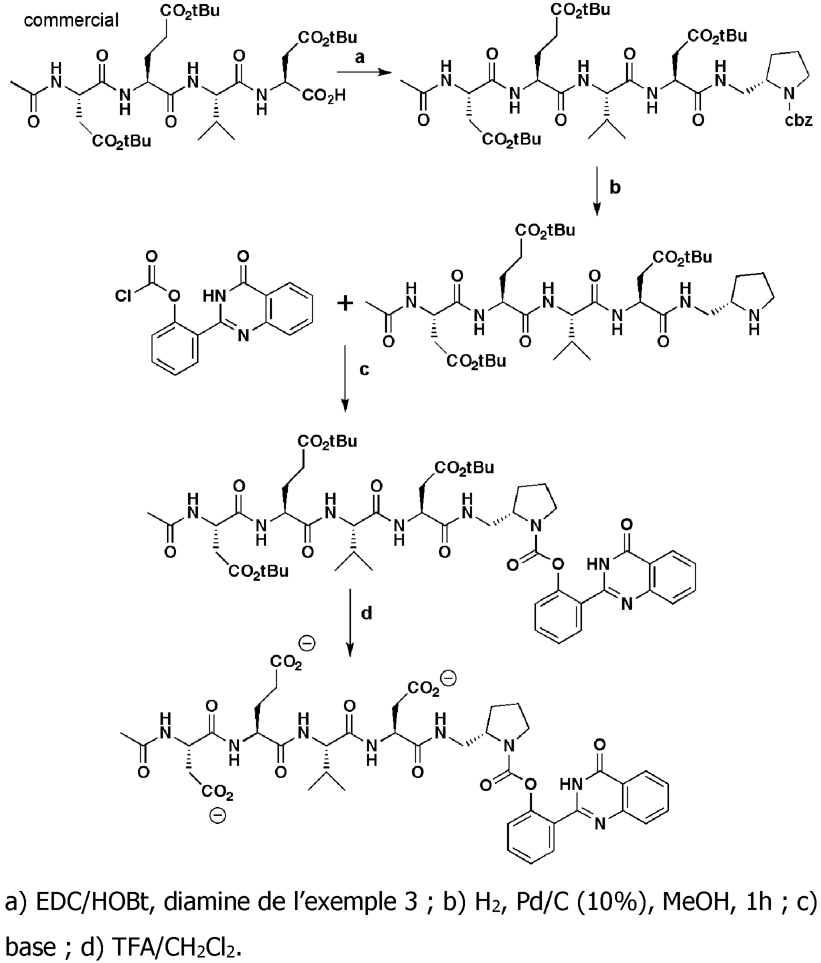

### Exemple I.11

La sonde **I.11** est synthétisée de manière analogue au composé **I.10** avec remplacement de la séquence peptidique protégée commerciale Ac-DEVD, par la séquence peptidique protégée commerciale Ac-HSSKLQ.

### Tests enzymatiques

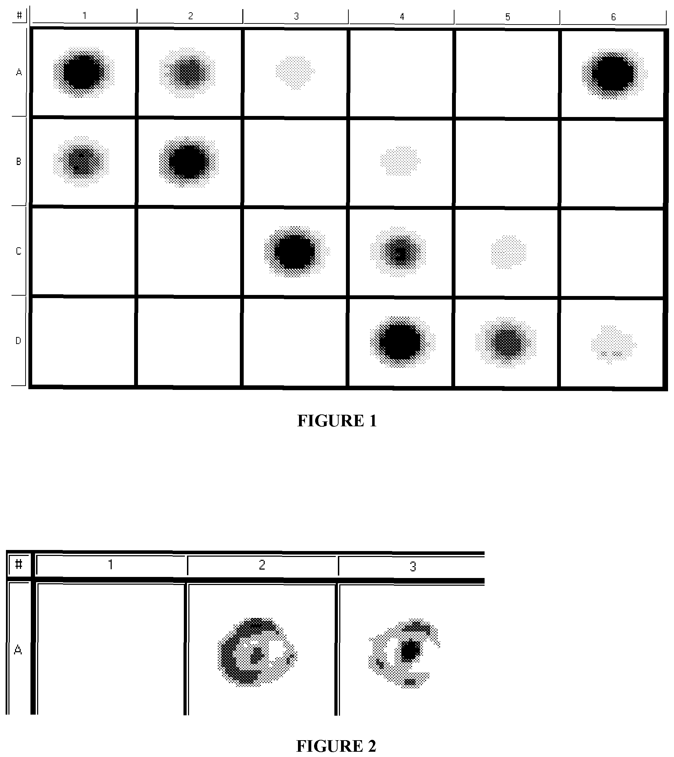

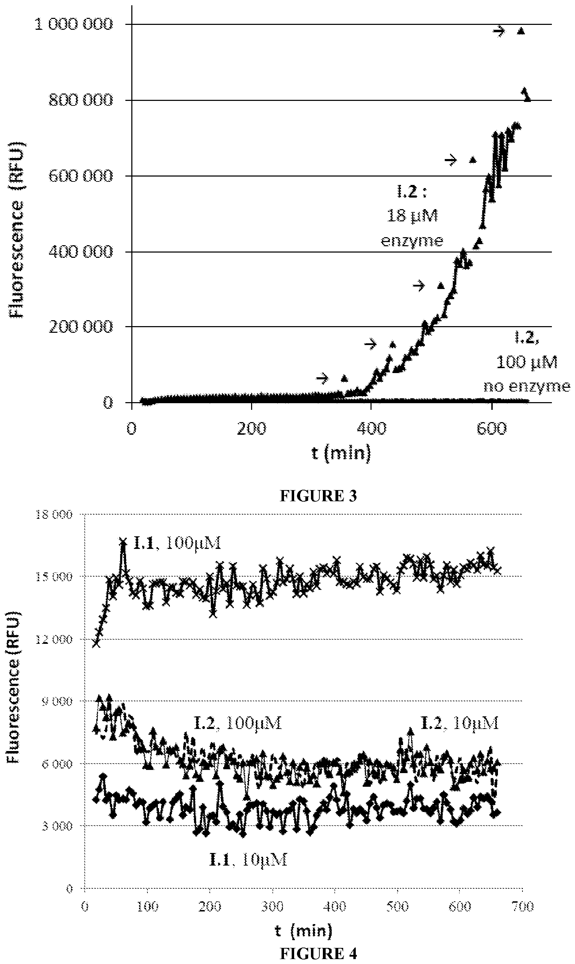

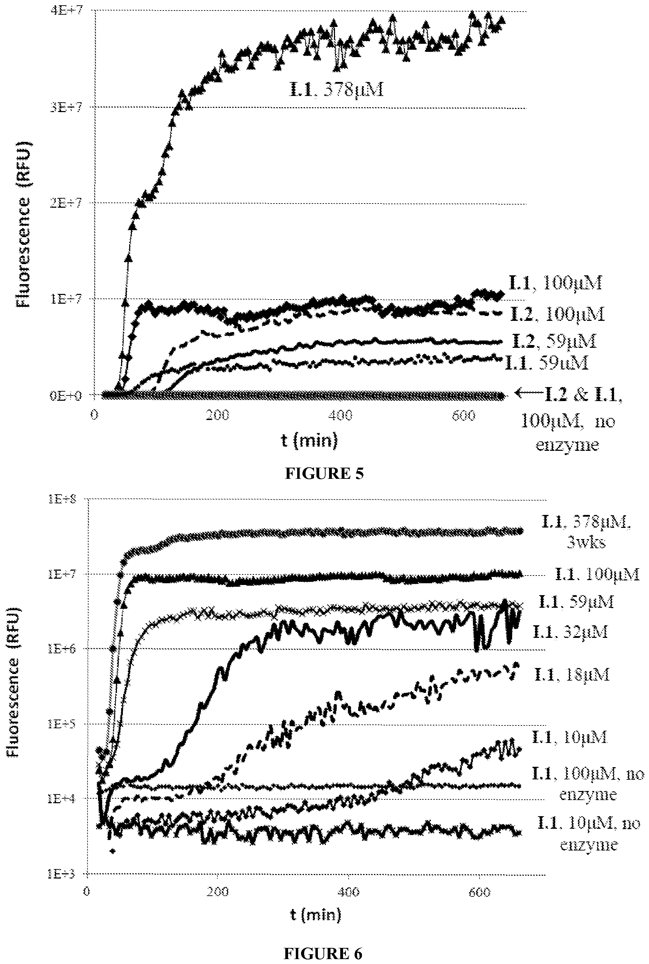

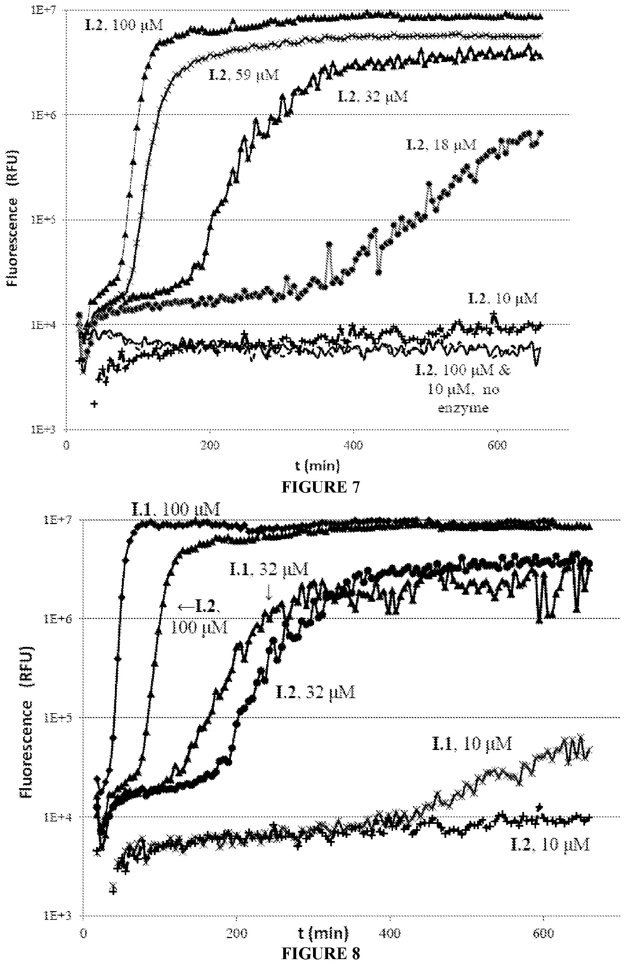

**Préambule :** Dans le cadre de l'invention, lors de la mise en oeuvre des tests enzymatiques, une fois la concentration minimale permettant de déclencher la nucléation de cristaux atteinte, un fluorophore solide peut se déposer d'abord sur les parois du réceptacle (un puits dans une plaque à multi-puits), avant de commencer à se déposer sur le fond du réceptacle. Le délai associé avec le déclenchement de la nucléation (≥ 10 min) se voit bien sur les courbes de cinétiques dans les figures présentées. Les tests enzymatiques effectués sont illustrés par les Figures annexées.
**La** **Figure 1** présente une image typique obtenue en mode scanning qui montre une visualisation spatiale de l'intensité de la fluorescence déclenchée par l'enzyme après 40 heures d'incubation, dans des puits d'une plaque à multi-puits (Corning), qui sont représentés dans une grille à 6 colonnes (1-6) et 4 rangées (A-D). Les puits A1, A6, B2, C3 et D4 contiennent de l'enzyme PLA avec une concentration initialement de 209 µM en sonde **I.1** ; les puits B1, A2, C4 et D5 contiennent de l'enzyme PLA avec une concentration initialement de 80 µM en sonde **I.1** ; les puits A3, B4, C5 et D6 contiennent de l'enzyme PLA avec une concentration initialement de 21,2 µM en sonde **I.1 ;** les autres puits ne contiennent pas de PLA mais contiennent soit 209 µM en sonde **I.1** (A4, B5, C6, D1, D3), soit 80 µM en sonde **I.1** (A5, B6, C1, D2), soit pas de sonde **I.1** (B3, C2). Le signal est représenté en échelle de gris, le noir correspondant à un maximum d'intensité.
**La** **Figure 2** présente l'image obtenue en mode scanning à haute résolution d'une autre expérience effectuée sur la sonde **I.1,** qui montre la tendance de la sonde activée par l'enzyme (puits A2 et A3) de précipiter à la fois dans le centre des puits mais aussi sur les rebords. Le puits A1 ne contient que le tampon. Le signal est représenté en échelle de gris, noir étant un maximum d'intensité.
**La** **Figure 3** présente les signaux G(t) correspondant à la fluorescence mesurée dans des puits dans le cas de la sonde **I.2** à une concentration de 18 µM en présence d'enzyme PLA et à une concentration de 100 µM sans enzyme. Pour une série (**I.2,** 18 µM, avec enzyme), la ligne en gras présente les données gardées après application de l'algorithme élaboré pour supprimer les cinq valeurs aberrantes (fléchés) du signal original (triangles non-liées).
**La** **Figure 4** montre l'évolution des signaux de fluorescence F(t) en fonction du temps, obtenus conformément au protocole décrit, en l'absence d'enzyme PLA dans des solutions aqueuses tamponnées à pH∼7.6, du composé **I.1** à 10 µM et à 100 µM, et du composé **I.2** à 10 µM et à 100 µM (ces deux derniers signaux sont presque entièrement superposés).
**La** **Figure 5** présente sur échelle linéaire les signaux F(t) obtenus conformément au protocole décrit, pour les composés **I.1** (à 378 µM, 100 µM et 59 µM) et **I.2** (à 100 µM et 59 µM) en présence d'enzyme PLA, et pour les mêmes composés à 100 µM chacun, mais sans PLA (contrôles).
**La** **Figure 6** présente sur échelle logarithmique les signaux F(t) obtenus conformément au protocole décrit, pour le composé **I.1** fraichement dissous à différentes concentrations 10-100 µM, en présence ainsi qu'en l'absence d'enzyme PLA, et pour ce même composé à 378 µM (calculé d'après sa concentration originale au moment de sa dilution) après 3 semaines de stockage en solution aqueuse (« 3 wks ») avec la PLA fraichement ajoutée.
**La** **Figure 7** présente sur échelle logarithmique les signaux F(t), obtenus conformément au protocole décrit, dans le cas du composé **I.2** fraichement dissous à différentes concentrations 10-100 µM, en présence ainsi qu'en l'absence d'enzyme PLA.
**La** **Figure 8** présente de manière comparée sur échelle logarithmique les signaux F(t) obtenus conformément au protocole décrit pour les composés **I.1** et **I.2** à des concentrations de 100, 32 et 10 µM en présence d'enzyme PLA.

Les sondes **I.1** et **I.2** ont été évaluées par incubation avec l'enzyme cible, la leucine aminopeptidase microsomale (EC 3.4.11.2; « PLA » ; commercial) dans un milieu *in vitro* dans des micro-plaques à multi-puits conçues pour des lecteurs à fluorescence. Les sondes ont été évaluées selon les critères suivants :
- Mise en évidence de l'intensité de fluorescence élevée générée par la présence de l'activité enzymatique (« allumé »),
- Mise en évidence de l'absence totale (« éteint ») de fluorescence dans des échantillons qui ne contiennent pas l'enzyme cible (pas de fluorescence intrinsèque),
- Mise en évidence de l'absence de toute dégradation hydrolytique de la sonde au cours du temps démontrant la robustesse des sondes à pH 7 dans un milieu aqueux (pas de signal faussement positif),
- Mise en évidence de la rapidité de réponse à la présence de l'activité enzymatique permettant d'atteindre rapidement un signal maximal,
- Mise en évidence de la corrélation du signal mesuré avec la concentration de la sonde,
- Mise en évidence de la forte photo-stabilité du fluorophore solide généré sous irradiation prolongée par le lecteur de fluorescence.

Les principaux tests ont été conduits à 25°C, dans des solutions tamponnées à pH 7,4 (pH physiologique). Des tests à 37°C auraient nécessité la protection des puits contre l'évaporation prématurée, par exemple avec la couverture par un film ce qui aurait réduit fortement la sensibilité de la mesure de fluorescence. Une température de 25°C a donc a été préférée.

### Réactifs utilisés :

Toutes les solutions finales pour les tests enzymatiques ont été tamponnées à un pH variant de 7,4 à 7,6 (en fonction de la température ambiante) avec un tampon de Tris/Tris.HCl à 25 mM (concentration totale), contenant 11 mM de NaCl. La leucine aminopeptidase microsomale (EC 3.4.11.2) du rein de cochon a été achetée chez Sigma (n° catalogue 069K7356) sous forme d'une suspension dans le sulfate d'ammonium (3,5 M) contenant MgCl₂ (10 mM), avec 3,5 mgmL⁻¹ de contenu en protéine et 10-40 U/mg de protéine, tel qu'il a été déterminé pour le substrat standard, le *p*-nitroanilide de leucyle, et a été stockée dans un frigo à 4 °C pour un maximum de 40 jours avant son utilisation. Cette suspension d'enzyme a été diluée initialement par un facteur de 100 à 200 dans du tampon Tris/Tris.HCl à 25 mM, homogénéisée, et laissée pendant une heure à température ambiante avant son utilisation, car l'incubation de l'enzyme comme décrit dans la littérature (eg. Beattie et al, Biochem. J. (1987) 242, 281-283) à 37 °C, avec ou sans MgCl₂ ou MnCl₂, n'avait pas démontré une réactivité conséquente supérieure. La concentration finale de l'enzyme dans les puits actifs a été ajustée à une valeur de 3,5 µgmL⁻¹. Les sondes **I.1** et **I.2** ont toujours été dissous, à partir des solides stockés dans des piluliers fermés hermétiquement, dans du MeOH (∼5 mgmL⁻¹) avant l'ajout du tampon Tris/Tris.HCl, avant de les diluer aux concentrations finales utilisées pour les tests enzymatiques.

### Analyses effectuées :

Les analyses ont été réalisées par la lecture des valeurs de fluorescence, mesurées en RFU, en fonction du temps. Les analyses ont été conduites dans des plaques en polypropylène noir à 96 (Corning, Corning Inc.) ou à 384 (Nunclon, Nunc Inc.) puits, et enregistrées sur un fluorimètre à microplaques (Mithras LB940 de Berthold Technologies). Ce fluorimètre a été utilisé dans un mode dit répété, où la fluorescence a été mesurée avec une lecture ici choisie de 3 secondes par puits, le faisceau d'excitation de 4mm de diamètre étant centré dans le puits. Malgré la tendance du fluorophore une fois libéré à se déposer dans un premier temps sur les parois du puits et non pas de tomber tout de suite au fond et au milieu du puits (voir **Figure 2**), les analyses en mode répété (ne considérant que la fluorescence des centres des puits) se sont montrées significatives. Il convient de noter que ce mode d'utilisation se rapproche du fonctionnement de High Throughput Screening optique par fluorescence, qui est une des applications envisagées pour ces types de sondes. Les images des **Figure 1** et **Figure 2** ont été acquises dans un mode dit scanning, où le faisceau excitant et le lecteur ont été déplacés, pas à pas, pour couvrir chaque puits y compris notamment ses bords. Dans ce mode scanning les puits ont été divisés dans une grille de lecture carrée, typiquement couvrant le puits avec une résolution de 8×8 à 15×15 pixels, chacun des pixels étant lus entre 0.1 et 0.3 s. Ce mode scanning élimine en partie les erreurs et le bruit de lecture de la fluorescence totale du puits que subit le mode répété, suite à la tendance du fluorophore une fois libéré de se déposer dans un premier temps sur les parois. Le mode scanning tel qu'il a été implémenté ici est aussi plus lent et moins adapté pour suivre qualitativement des cinétiques de réaction. Il convient de noter que ce mode d'utilisation se rapproche du fonctionnement de Cell Counting, qui est une deuxième application envisagée pour ces types de sondes.

On remarque sur la **Figure 1** que la fluorescence observée varie en fonction de la concentration initiale dans chaque puits (les trois suites de puits, avec une concentration initiale en sonde **I.1** de 209 µM, 80 µM et 21 µM, sont facilement repérables), et qu'il n'y a pas de fluorescence comparable dans les autres puits (les puits contenant de la sonde sans enzyme, à 209 µM et à 80 µM, sont aussi invisibles que les puits ne contenant pas de sonde), ce qui illustre la fonctionnalité éteint-ALLUME de la sonde, l'absence de toute fluorescence intrinsèque de la sonde, et l'absence de tout signal faussement positif dû à l'hydrolyse spontanée de la sonde, même après les 40 heures d'incubation.

La longueur d'onde d'excitation pour les sondes **I.1** et **I.2** (à base d'HPQ) a été fixée à 355 nm, et la longueur d'onde d'émission repérée a été fixée à 510 nm , car le fluorimètre utilisé dispose des filtres de type bandpass pour sélectionner ces longueurs d'onde avec une largeur de bande de 40 nm pour le faisceau d'excitation et de 10 nm pour celui de l'émission.

### Protocole des acquisitions :

L'enzyme a été ajoutée en dernier aux puits préparés. Après ajout de l'enzyme, le contenu du puits a été aspiré et relâché deux fois avec une pipette, afin d'homogénéiser autant que possible le mélange. Ceci a engendré un délai de 5 à 20 minutes avant la première acquisition, en fonction du nombre de puits par acquisition, mais ce délai n'a pas empêché l'acquisition au moment où les premiers puits ont commencé à montrer de la fluorescence. L'ajout de l'enzyme puits par puits a été fait en respectant le délai de lecture entre les puits, afin que les temps de réaction de chaque puits soient les mêmes pour chaque lecture de la plaque pour permettre la comparaison des données acquises pour des puits différents (dans ces expériences il y avait entre 3 secondes et 20 secondes de délai entre la lecture des puits voisins, à multiplier par jusqu'à 77 puits lus par acquisition à un temps *t*). Sauf mention contraire, des valeurs de fluorescence A_{X,i}(t) d'au moins cinq puits (répétitions *i*) pour chaque suite *X* de conditions actives (concentration et nature du substrat, concentration en enzyme, ...) et au moins trois répétitions pour chaque suite de conditions contrôles (par exemple, pas d'enzyme et/ou pas de substrat) ont été mesurées dans chaque protocole. Les plaquettes ont été agitées entre les lectures pendant environ 10 secondes.

### Pre-traitement des données:

Le mode répété acquiert une fonction de valeurs de fluorescence de chaque puits, mesurée en RFU en fonction du temps ; ces fonctions de valeurs brutes sont notées A_{X,i}(t) (les indices indiquant la répétition de la suite *X*). La moyenne, B(t), des valeurs de fluorescence brutes (en RFU) obtenues pour la suite de (trois ou plus) puits contrôles contenant la solution tamponnée et une quantité d'enzyme égale à celle utilisée dans les puits actifs, mais sans substrat, est calculée. Puis les fonctions G*_{X,i}(t) = A_{X,i}(t) - B(t) ont été calculées, pour éliminer un signal environnemental superposé sur le signal mesuré provenant, entre autres facteurs, de la dispersion de la lumière par la plaquette, l'enzyme et le milieu tamponné, de la dispersion de la lumière par la poussière présente dans l'air et sur la surface des puits. Puis, les G*_{X,i}(t) ont été normalisés pour donner les fonctions G_{X,i}(t) = G*_{X,i}(t) × B(t=0) / B(t) pour chaque puits. Cette normalisation a permis une comparaison des valeurs de fluorescence au cours du temps, en compensant un peu les effets tels que le déplacement de la calibration du détecteur (« detector drift ») ou des variations éventuelles dans l'intensité émise de la lampe UV au cours des acquisitions qui pouvaient durer plus de 24 heures.

**La** **Figure 3** met en évidence que la sonde **I.2** ne se dégrade pas en absence d'enzyme (absence de tout signal faussement positif), même à une concentration élevée, mais qu'elle permet bien de générer un signal très fort en présence d'enzyme, et ceci même à une concentration bien plus faible.

### Données aberrantes :

Les données brutes de chaque puits ont été examinées pour étudier leur cohérence, car il a parfois été constaté qu'un point (points désignés par une flèche sur la **Figure 3**) était très différent de ses voisins, par exemple jusqu'à 300% de RFU en plus ou en moins que la moyenne arithmétique des valeurs voisines. Il a été considéré dans des cas pareils qu'il ne s'agissait pas de variations naturelles du signal acquis, mais de valeurs aberrantes dues par exemple à la présence de particules de poussière qui seraient arrivées sur la plaque d'analyse dispersant la lumière, et qui auraient été balayées par les courants d'air près du lecteur. Il a donc été décidé d'abandonner ces points considérés comme aberrants, tout en conservant la courbe totale obtenue pour le puits concerné. Un test a été élaboré, afin de déterminer si un point pouvait être qualifié d'aberrant ou non. Pour un point G_{x,i}(t), une valeur attendue, inversement pondérée par la grandeur des valeurs voisines a été calculée pour donner µ_{X,i}(t) = [(3G_{X,i}(t-3) + 2G_{X,i}(t-2) + 2G_{X,i}(t+2) + 3G_{X,i}(t+3))/10]. Ensuite, la valeur Δ_{X,i}(t) =[G_{x,i}(t) - M_{X,i}(t)]²/(|µ_{X,i}(t)) + |G_{x,i}(t)l) a été calculée. Les points G_{x,i}(t) correspondants aux valeurs les plus grandes de Δ_{X,i}(t) ont pu être rejetés en ordre de décroissance, et les valeurs de µ_{X,i}(t') des points voisins ont été recalculés jusqu'à ce que 5% des points obtenus d'une série soient rejetés (par exemple, les cinq points fléchés sur la **Figure 3**). Cette méthode a été adaptée pour les valeurs vers la fin de l'acquisition (qui devaient correspondre à un plateau de fluorescence) mais n'a pas été appliquée aux trois points de données initiales, par crainte de masquer une croissance rapide du signal correspondant à l'activation de la sonde. Cette méthode n'a pas non plus été appliquée aux régions de données dans lesquelles les valeurs de fluorescence correspondaient à une augmentation non linéaire rapide. La courbe obtenue par cette méthode, appliquée à la courbe G(t) avec les points de données en triangles, est également représentée sur la **Figure 3** en gras, mettant en évidence les points (pointés d'une flèche) qui ont été éliminés.

### Données retenues et obtention des valeurs moyennes de fluorescence F_{X}(t) :

Pour analyser les données les plus cohérentes, les courbes de données sans points aberrants, G_{X,i}(t), de jusqu'à deux des répétitions *i* de chaque suite *X* ont été rejetées, avec les conditions que (1) au moins trois répétitions pour chaque suite seraient toujours gardées et (2) le signal d'un puits rejeté ait franchi un seuil arbitraire d'écart entre ses valeurs et les valeurs de la moyenne globale de la suite M_{X}(t). Pour évaluer une rejection, la moyenne Q_{X,i} de toutes les valeurs de la fonction |(G_{X,i}(t) - M_{X}(t))²/M_{X}(t)| a d'abord été calculée; pour rejeter une répétition *i*, le rapport Rᵢ = [Q_{X,i}/(la valeur moyenne des cinq derniers points de M(t))] devait être supérieur à 0,025. Après avoir ainsi rejeté pour chaque suite *X* le puits *i* avec le plus grand Q_{X,i}, les M_{X}(t) et Q_{X,i} ont été recalculés pour réévaluer les Q_{X,i} restants, et la procédure réappliquée. A noter que cette mesure ne lutte pas intrinsèquement contre des variations éventuelles entre des valeurs voisines, G_{X,i}(t) et G_{X,i}(t') (cette différence est comparable à un rapport signal/bruit). Les répétitions gardées sont notées « acceptables » et pour chaque suite *X* de points gardée, les valeurs G_{X,i}(t) des fonctions retenues ont été moyennées, afin de donner les séries de valeurs de fluorescence Fₓ(t) de chaque suite de conditions *X*.

### Données acquises :

Une acquisition pendant 11 heures en mode répétée a été entreprise (1) pour démontrer la stabilité des sondes **I.1** et **I.2,** (2) pour mettre en évidence leurs valeurs minimales de fluorescence de fond, (3) pour mettre en évidence les signaux importants déclenchés par l'activité enzymatique, avec une gamme variable de concentrations, (4) pour obtenir la cinétique approximative d'action des sondes, et (5) pour évaluer en fonction du temps écoulé, si les signaux obtenus dus à l'activité enzymatique pouvaient être considérés comme quantitatifs avec ce mode d'acquisition rapide. Chacune des sondes a été testées dans une gamme de concentrations allant de 10 µM à 100 µM ; pour chacune de ces sondes deux séries de contrôles de la stabilité ont été mesurées, une à 100 µM et une à 10 µM, pour démontrer l'absence de tout signal faussement positif. Une série de mesures de contrôle de la fluorescence de fond a été effectuée, avec des puits contenant de l'enzyme dans le tampon standard sans aucune sonde. Une dernière série de mesures a été menée en utilisant une solution de sonde **I.1** préparée trois semaines auparavant (« 3wks »), pour donner une autre estimation de la robustesse de cette sonde envers l'hydrolyse spontanée dans la pratique. Chaque lecture a duré 3 secondes par puits. Les résultats obtenus sont présentés sous format graphique, sur les **Figures 4** à **8****.**

La **Figure 4** met en évidence la stabilité totale à l'hydrolyse spontanée des substrats selon l'invention et l'absence de tout signal faussement positif pour les sondes **I.1** et **I.2** pendant les 11h d'acquisition. Pour comparaison, des sondes précédemment publiées par l'un des inventeurs (Zhang, Waibel et Hasserodt, Chem. Eur. J. 2010, 16, 792) montraient, dans des conditions similaires pour une sonde à 38 µM, une augmentation de signal des puits contrôles en l'absence d'enzyme de l'ordre de 10-15% pendant 150 minutes (donc un signal faussement positif). Avec les sondes selon l'invention, AUCUNE augmentation de signal par rapport au bruit des signaux n'est constatée même à une concentration de 100 µM et pendant 660 minutes.

Les valeurs de fluorescence intrinsèque pour la sonde **I.2** (contre-ion utilisé : CF₃CO₂⁻) semblent insensibles à sa concentration en solution, tandis que les valeurs de fluorescence pour la sonde **I.1** (contre-ion utilisé : pTsO⁻) montrent une certaine dépendance sur sa concentration.

La **Figure 5** met en évidence le signal intense de fluorescence qui est rapidement obtenu en présence de l'enzyme, avec les composés **I.1** et **I.2** à différentes concentrations, et montre la faiblesse des valeurs de fluorescence intrinsèque comparées à celles-ci (observées pour les sondes en l'absence d'enzyme). Il convient de noter que le délai pour obtenir une augmentation intense du signal est très faible : il est d'au moins 10 minutes pour les raisons exposées dans le préambule mais est, en général, inférieur à 100 minutes.

La **Figure 6** met en évidence l'obtention du signal intense de fluorescence rapidement obtenu avec le composé **I.1** en présence de l'enzyme, alors que de très faibles valeurs de fluorescence de fond sont observées pour la même sonde en l'absence d'enzyme. Il convient de souligner qu'à partir de 500 minutes, même avec la plus faible concentration de 10 µM en composé **I.1,** on obtient un signal qui ne peut pas être confondu avec la fluorescence de fond même obtenue pour la série de mesures effectuées à 100 µM, correspondant à *dix fois* sa concentration en sonde mais sans enzyme pour l'activer. Par ailleurs, les signaux, une fois le plateau atteint, ne baissent pas avec le temps : les taux d'irradiation de l'instrument ne suffisent donc pas à causer une photo-dégradation repérable.

Les résultats présentés sur les courbes de la **Figure 7** dans le cas du composé **I.2** sont comparables, et donc aussi satisfaisants.

La **Figure 8** montre les différences de cinétique dans le déclenchement du signal pour les sondes **I.1** et **I.2** en fonction de la concentration. On notera que les valeurs des plateaux de fluorescence pour les deux sondes comparées à concentrations identiques (100 µM et 32 µM) sont les mêmes, comme attendu pour une activation totale de la sonde présente dans le puits.

Les temps t_{1/2} observés dans le protocole utilisé pour les sondes **I.1** et **I.2** incubées avec l'enzyme correspondant à la moitié des signaux maximaux observés pendant les 11h d'acquisition ont été calculés et sont présentés dans le **Tableau 2.** Ces valeurs donnent une estimation de la rapidité d'action des sondes avec les faibles concentrations en enzyme utilisées.

**Tableau 2**

| | Valeurs t_{1/2} (min) |
|---|---|
| **I.1,** 378 µM «3wks » | 71 |
| **I.1,** 100 µM | 55 |
| **I.2,** 100 µM | 125 |
| **I.1,** 59 µM | 93 |
| **I.2,** 59 µM | 162 |
| **I.1,** 32 µM | 237 |
| **I.2,** 32 µM | 344 |
| **I.1,** 18 µM | 499 |
| **I.2,** 18 µM | 460 |
| **I.1,** 10 µM | 520 |
| **I.2,** 10 µM | 533 |

En utilisant les valeurs de fluorescence « de plateau » (maximales et ne changeant pas avec le temps) mesurées avec des concentrations en sonde de 378 µM à 32 µM (après 200-400 min jusqu'à la fin de l'acquisition), le rapport signalₚₗₐₜₑₐᵤ/concentration pour chaque série de puits a été évalué. Une variation de l'ordre de seulement 20% a été constatée entre les valeurs de rapport obtenues pour des séries de mesures différentes, ce qui met en évidence une corrélation entre le signal mesuré pour les tests avec l'enzyme, et la concentration initiale de la sonde, quand la partie fluorophore de ces sondes est le même. En utilisant la valeur moyenne des rapports obtenus, la concentration initiale des sondes dans les puits actifs a été estimée. Les valeurs obtenues sont présentées dans le **Tableau 3.** Les valeurs ainsi estimées sont assez proches des valeurs réelles. Dans le cas des concentrations à 18 µM et à 10 µM, les plateaux n'ayant pas été atteints, les concentrations calculées se sont montrées, comme attendues, sous-estimées.

**Tableau 3**

| | Signal / Conc (RFU µM⁻¹) | ***Canc prédit (µM)*** |
|---|---|---|
| **I.1,** 378 µM, 3 sem. | 98684 | ***419*** |
| **I.1,** 100 µM | 95711 | ***108*** |
| **I.2,** 100 µM | 87269 | ***98*** |
| **I.1,** 59 µM | 63054 | ***42*** |
| **I.2,** 59 µM | 95096 | ***63*** |
| **I.1,** 32 µM | 72794 | ***26*** |
| **I.2,** 32 µM | 110289 | ***40*** |
| **I.1,** 18 µM | | ***6*** |
| **I.2,** 18 µM | | ***6*** |
| **I.1,** 10 µM | | ***0*** |
| **I.2,** 10 µM | | ***1*** |
| **Rapport Moyen** | 88985 | |

## Revendications

1. Substrat de peptidase de formule (I) : 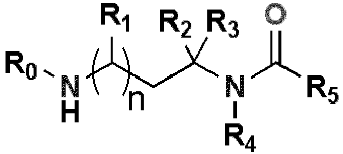 dans laquelle :
- R₀ est un groupement peptidyle ou aminoacide lié au groupement NH via son extrémité carboxy-terminale, capable d'être clivé du reste du substrat par la peptidase cible,
- n est 0 ou 1,
- R₁ est un atome d'hydrogène ou un groupe méthyle, iso-propyle, iso-butyle ou benzyle,
- R₅ est un dérivé phénoxy de formule (A) 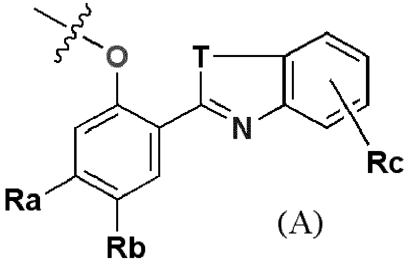 dans laquelle :
∘ T est -NH-C(O)-, -S-, -O-, -NH, N-alkyle ou N-aryle,
∘ Ra est l'hydrogène ou un substituant -CN ou -COORd, avec Rd qui représente un groupe (C₁-C₄)alkyle, ou bien Ra est -CONReRf, avec Re et Rf, identiques ou différents, qui représentent l'hydrogène ou un groupe (C₁-C₄)alkyle, ou bien Ra est -CF₃, ou un 2-oxazolyle, 2-thiazolyle, 2-imidazolyle (benzo-condensé ou pas), 4-pyrimidinon-2-yle ou quinazolinon-2-yle,
∘ Rb est l'hydrogène, un atome de chlore, -OH, -NH₂, -NRgRh ou - ORg, avec Rg et Rh identiques ou différents, qui représentent un groupe (C₁-C₄)alkyle,
∘ ou bien Ra et Rb sont liés entre eux pour former une chaine hydrocarbonée comprenant 4 ou 5 chainons, saturée ou insaturée, substituée ou non substituée, éventuellement interrompue par un ou plusieurs hétéroatomes choisis parmi N, S et O,
∘ Rc est l'hydrogène, Br, Cl, I ou F,
- R₂, R₃ et R₄ sont définis comme suit :
∘ soit R₂=H et R₃ et R₄ sont liés entre eux et forment un enchaînement -(CH₂)m- avec m = 3, 4 ou 5,
∘ soit R₂, R₃ et R₄, identiques ou différents, représentent un groupe (C₁-C₄)alkyle,
sous la forme d'un mélange d'isomères optiques ou de diastéréoisomères selon toutes proportions, ou sous une forme enrichie en un isomère optique ou en un diastéréoisomère.

2. Substrat de peptidase selon la revendication 1 **caractérisé en ce que** R₂, R₃ et R₄, identiques ou différents, représentent un méthyle ou un éthyle.

3. Substrat de peptidase selon la revendication 2 **caractérisé en ce que** R₂=R₃=R₄=-CH₃.

4. Substrat de peptidase selon l'une quelconque des revendications 1 à 3 de formule (IA) : 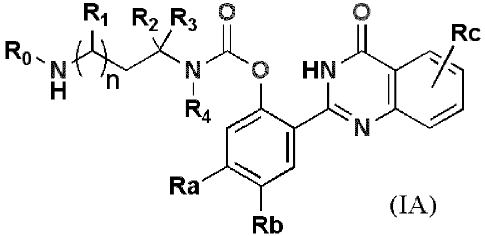 où Ro, R₁, R₂, R₃, R₄, n, Ra, Rb et Rc sont tels que définis aux revendications 1 à 3, sous la forme d'un mélange d'isomères optiques ou de diastéréoisomères selon toutes proportions, ou sous une forme enrichie en un isomère optique ou en un diastéréoisomère.

5. Substrat de peptidase selon l'une des revendications 1 à 4 **caractérisé en ce que** Ra=Rb=Rc=H ou Ra=H, Rb=Rc=Cl.

6. Substrat de peptidase selon l'une des revendications 1 à 4 **caractérisé en ce que** Ra et Rb sont liés entre eux, de sorte que le substrat selon l'invention répond à la formule (IA') ou (IB') : 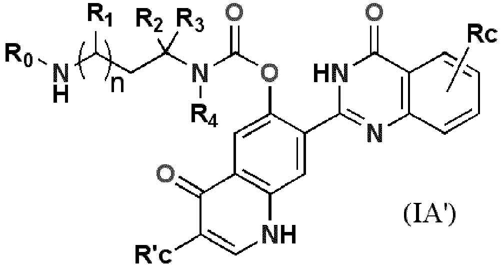 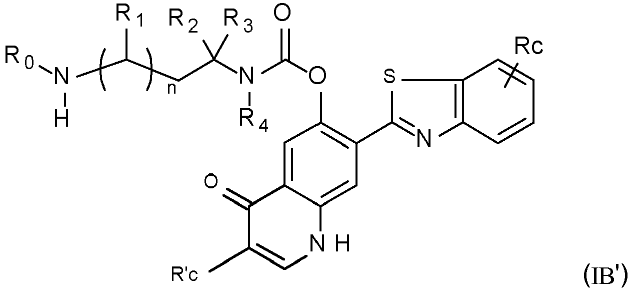 où n, R₀, R₁, R₂, R₃, R₄ et Rc sont tels que définis aux revendications 1 à 3 et R'c représente un atome d'hydrogène ou un groupe -COO(C₁-C₄)alkyle, sous la forme d'un mélange d'isomères optiques ou de diastéréoisomères selon toutes proportions, ou sous une forme enrichie en un isomère optique ou en un diastéréoisomère.

7. Substrat de peptidase selon l'une quelconque des revendications 1 à 6 **caractérisé en ce que** R₀ représente un aminoacide ou un groupement peptidyle comportant au plus 10 aminoacides dans lequel les aminoacides sont identiques ou différents choisis parmi les aminoacides naturels, et l'extrémité N-terminale peut être non substituée ou substituée par un groupe acyle - COR", R" étant un groupe (C₁-C₆)alkyle ou un groupe O-(C₁-C₆)alkyle.

8. Substrat de peptidase selon l'une quelconque des revendications 1 à 7 **caractérisé en ce que** n=0.

9. Substrat de peptidase selon l'une quelconque des revendications 1 à 8 **caractérisé en ce que** n=1 et R₁ représente un groupe méthyle, iso-propyle, iso-butyle ou benzyle.

10. Substrat de peptidase selon l'une quelconque des revendications 1 à 9 **caractérisé en ce que** le groupement peptidyle ou aminoacide R₀ est un substrat pour la leucine aminopeptidase, la caspase-3, la peptidase de VIH-1, la rénine, la thrombine, la tryptase, la cathepsine K, les ACE plasmepsines I ou II, la β-sécrétase ou l'antigène spécifique à la prostate « PSA ».

11. Substrat de peptidase selon la revendication 1 choisi parmi les composés de formule :

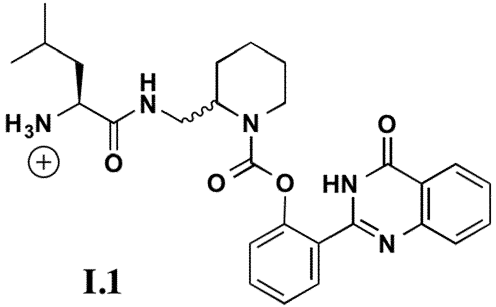

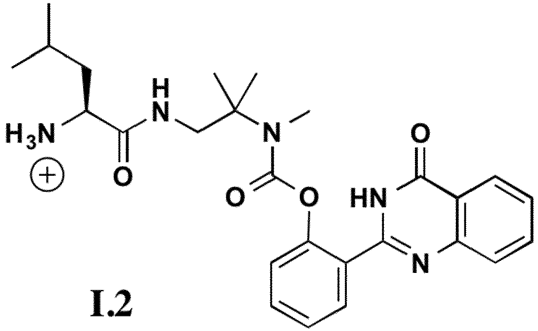

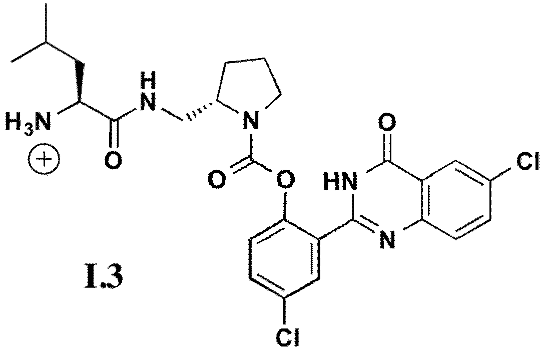

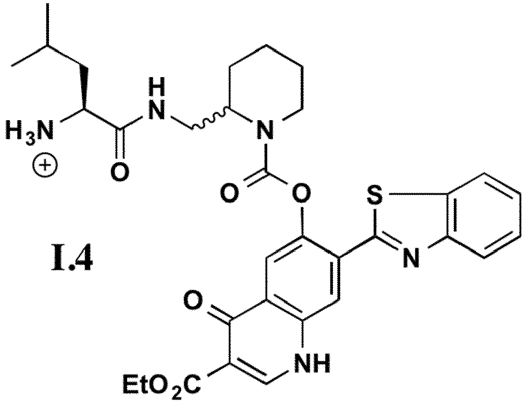

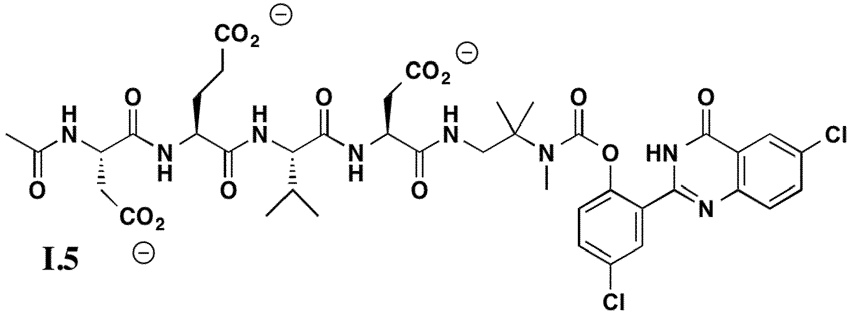

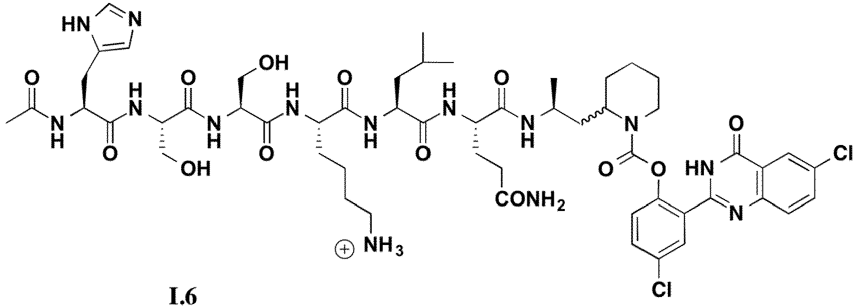

12. Procédé pour détecter la présence d'une peptidase catalytiquement active comprenant les étapes de :
- mise en contact d'un échantillon suspecté de contenir ladite peptidase avec un substrat selon l'une quelconque des revendications 1 à 11 ;
- application de conditions appropriées pour permettre la formation d'un précipité fluorescent par clivage de la liaison covalente entre NH et R₀, suivi d'un clivage de la liaison -C(O)-R₅ suite à une cyclisation de l'espaceur ; et
- analyse quantitative ou qualitative dudit précipité fluorescent.

13. Procédé selon la revendication 12 où l'analyse du précipité fluorescent comprend les étapes :
- d'exposition du précipité fluorescent à une source lumineuse capable de produire de la lumière à une longueur d'onde d'absorption du précipité fluorescent ; et
- de détection de la fluorescence du précipité résultante.

## Patentansprüche

1. Peptidasesubstrat mit der Formel (I): 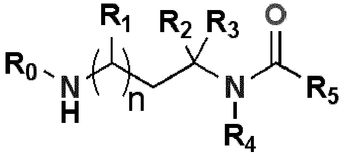 wobei:
- R₀ eine Peptidyl- oder Aminosäuregruppe ist, die über ihr carboxyterminales Ende mit der NH-Gruppe verbunden ist und durch die Zielpeptidase vom Rest des Substrats gespalten werden kann,
- n 0 oder 1 ist,
- R₁ ein Wasserstoffatom oder eine Methyl-, Iso-Propyl-, Iso-Butyl- oder Benzylgruppe ist,
- R₅ ein Phenoxy-Derivat mit der Formel (A) ist, 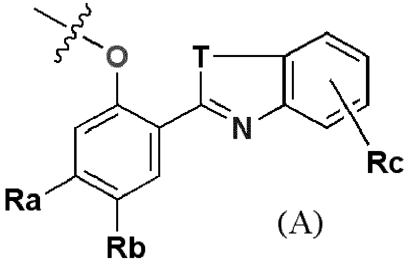
wobei:
∘ T -NH-C(O)-, -S-, -0-, -NH, N-Alkyl oder N-Aryl ist,
∘ Ra der Wasserstoff oder ein Substituent -CN oder -COORd, ist mit Rd, das eine (C₁-C₄)Alkylgruppe darstellt, oder auch Ra -CONReRf ist, mit Re und Rf, die identisch oder unterschiedlich sind, die Wasserstoff oder eine (C₁-C₄)Alkylgruppe darstellen, oder auch Ra -CF₃ ist, oder ein 2-Oxazolyl, 2-Thiazolyl, 2-Imidazolyl (benzoangebunden oder nicht), 4-Pyrimidinon-2-yl oder Chinazolinon-2-yl,
∘ Rb Wasserstoff, ein Chloratom, -OH, -NH₂, -NRgRh oder -ORg ist, mit Rg und Rh identisch oder unterschiedlich, die eine (C₁-C₄)Alkylgruppe darstellen,
∘ oder auch Ra und Rb miteinander verbunden sind, um eine Kohlenwasserstoffkette mit 4 oder 5 Kettengliedern zu bilden, die gesättigt oder ungesättigt, substituiert oder unsubstituiert ist, eventuell unterbrochen durch ein oder mehrere Heteroatome, die unter N, S und 0 ausgewählt sind,
∘ Rc Wasserstoff, Br, Cl, I oder F ist,
- R₂, R₃ und R₄ wie folgt definiert sind:
∘ Entweder R₂=H und R₃ und R₄ miteinander verbunden sind und bilden eine Sequenz -(CH₂)m- mit m=3, 4 oder 5,
∘ oder R₂, R₃ und R₄, identisch oder unterschiedlich, stellen eine (C₁-C₄)Alkylgruppe,
in Form einer Mischung aus optischen Isomeren oder Diastereoisomeren in beliebigem Verhältnis oder in einer mit einem optischen Isomer oder Diastereoisomer angereicherten Form.

2. Peptidasesubstrat nach Anspruch 1, **dadurch gekennzeichnet, dass** R₂, R₃ und R₄, identisch oder unterschiedlich, ein Methyl oder ein Ethyl darstellen.

3. Peptidasesubstrat nach Anspruch 2, **dadurch gekennzeichnet, dass** R₂=R₃=R₄=-CH₃.

4. Peptidasesubstrat nach einem der Ansprüche 1 bis 3 mit der Formel (IA): 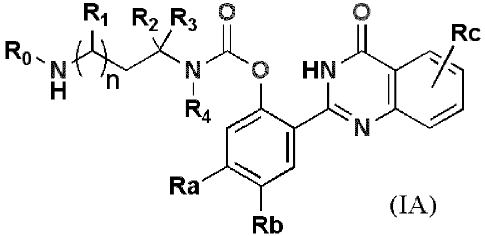 wobei R₀, R₁, R₂, R₃, R₄, n, Ra, Rb und Rc so wie in den Ansprüchen 1 bis 3 definiert sind, in Form einer Mischung aus optischen Isomeren oder Diastereoisomeren in beliebigem Verhältnis oder in einer mit einem optischen Isomer oder Diastereoisomer angereicherten Form.

5. Peptidasesubstrat nach einem der Ansprüche 1 bis 4, **dadurch gekennzeichnet, dass** Ra=Rb=Rc=H oder Ra=H, Rb=Rc=Cl.

6. Peptidasesubstrat nach einem der Ansprüche 1 bis 4, **dadurch gekennzeichnet, dass** Ra und Rb miteinander verbunden sind, so dass das erfindungsgemäße Substrat der Formel (IA') oder (IB') entspricht: 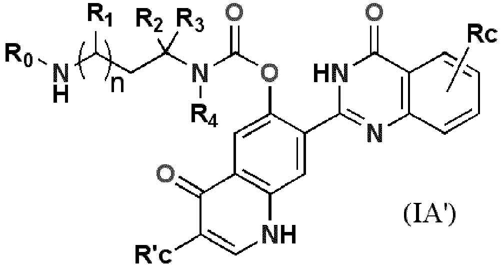 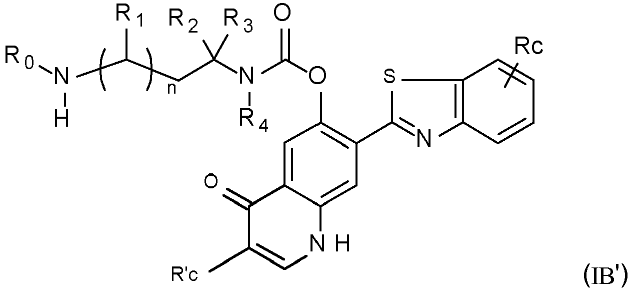 wobei n, R₀, R₁, R₂, R₃, R₄ und Rc so wie in den Ansprüchen 1 bis 3 definiert sind und R'c ein Wasserstoffatom oder eine -coo(C₁-C₄)Alkylgruppe darstellt, in Form einer Mischung aus optischen Isomeren oder Diastereoisomeren in beliebigem Verhältnis oder in einer mit einem optischen Isomer oder Diastereoisomer angereicherten Form.

7. Peptidasesubstrat nach einem der Ansprüche 1 bis 6, **dadurch gekennzeichnet, dass** R₀ eine Aminosäure oder eine Peptidylgruppe darstellt, die höchstens 10 Aminosäuren enthält, wobei die Aminosäuren identisch oder unterschiedlich und unter den natürlichen Aminosäuren gewählt sind, und das N-terminale Ende unsubstituiert oder durch eine Acylgruppe - COR" substituiert sein kann, wobei R" eine (C₁-C₆)Alkylgruppe oder eine O-(C₁-C₆)Alkylgruppe ist.

8. Peptidasesubstrat nach einem der Ansprüche 1 bis 7, **dadurch gekennzeichnet, dass** n=0.

9. Peptidasesubstrat nach einem der Ansprüche 1 bis 8, **dadurch gekennzeichnet, dass** n=1 und R₁ eine Methyl-, Iso-Propyl-, Iso-Butyl- oder Benzylgruppe darstellt.

10. Peptidasesubstrat nach einem der Ansprüche 1 bis 9, **dadurch gekennzeichnet, dass** die Peptidyl- oder Aminosäuregruppe R₀ ein Substrat für Aminopeptidaseleucin, Caspase-3, HIV-1-Peptidase, Renin, Thrombin, Tryptase, Cathepsin K, ACE-Plasmepsine I oder II, β-Sekretase oder das prostataspezifische Antigen "PSA" ist.

11. Peptidasesubstrat nach Anspruch 1, das unter Verbindungen folgender Formel gewählt ist:

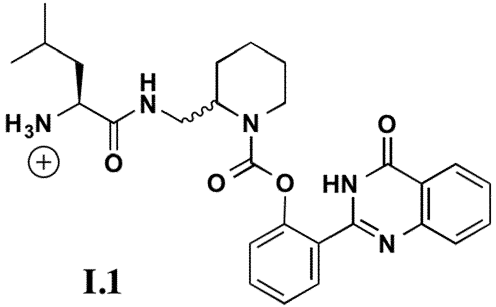

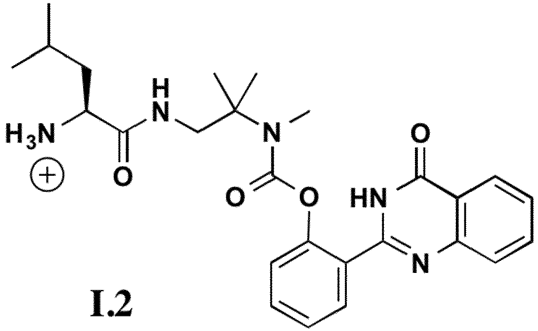

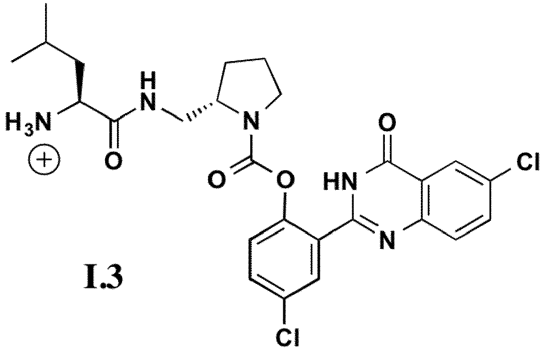

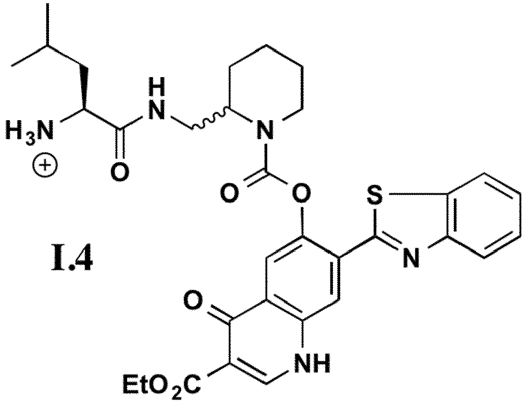

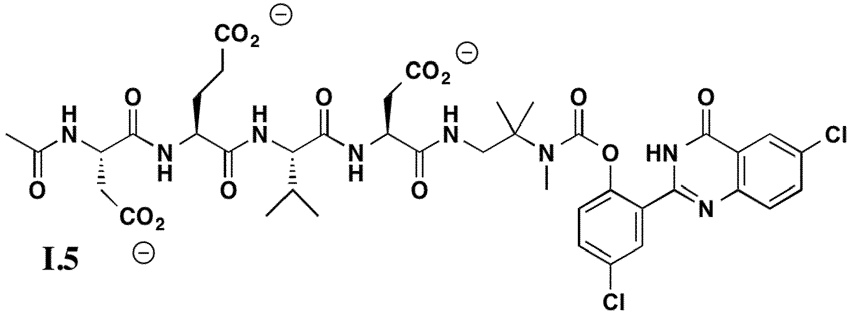

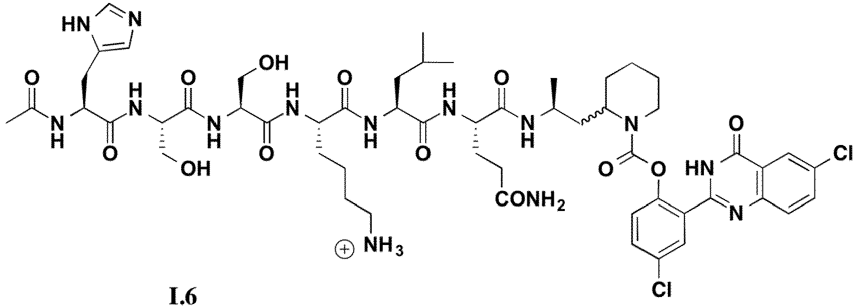

12. Verfahren zur Erkennung einer vorhandenen katalytisch aktiven Peptidase, das folgende Schritte umfasst:
- Zusammenführung einer Probe, von der vermutet wird, dass sie die Peptidase enthält, mit einem Substrat nach einem der Ansprüche 1 bis 11;
- Anwendung geeigneter Bedingungen, um die Bildung eines fluoreszierenden Niederschlags durch Spalten der kovalenten Bindung zwischen NH et R₀ zu ermöglichen mit anschließender Spaltung der -C(O)-R₅ Verbindung nach einer Zyklisierung des Spacers; und
- Quantitative oder qualitative Analyse des fluoreszierenden Niederschlags.

13. Verfahren nach Anspruch 12, wobei die Analyse des fluoreszierenden Niederschlags die folgenden Schritte umfasst:
- Exposition des fluoreszierenden Niederschlags gegenüber einer Lichtquelle, die Licht mit einer Absorptionswellenlänge des fluoreszierenden Niederschlags erzeugen kann; und
- Erkennung der Fluoreszenz des resultierenden Niederschlags.

## Claims

1. A peptidase substrate of formula (I): 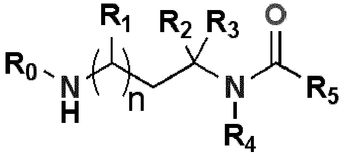 wherein:
- R₀ is a peptidyl or amino acid group bound to the NH group via its carboxy-terminal end, capable of being cleaved from the remainder of the substrate by the target peptidase,
- n is 0 or 1,
- R₁ is a hydrogen atom or a methyl, iso-propyl, iso-butyl or benzyl group,
- R₅ is a phenoxy derivative of formula (A) 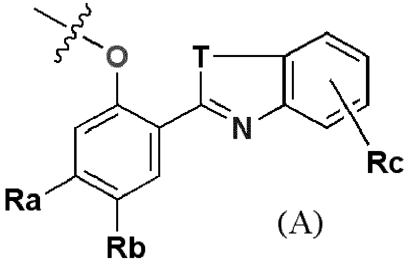
wherein:
∘ T is -NH-C(O)-, -S-, -0-, -NH, N-alkyl or N-aryl,
∘ Ra is hydrogen or a substituent -CN or -COORd, with Rd which represents a (C₁-C₄)alkyl group, or else Ra is -CONReRf, with Re and Rf, either identical or different, which represent hydrogen or a (C₁-C₄) alkyl group, or else Ra is -CF₃, or a 2-oxazolyl, 2-thiazolyl, 2-imidazolyl (either benzo-fused or not), 4-pyrimidinon-2-yl or quinazolinon-2-yl,
∘ Rb is hydrogen, a chlorine atom, -OH, -NH₂, -NRgRh or -ORg, with Rg and Rh either identical or different, which represent a (C₁-C₄)alkyl group,
∘ or else Ra and Rb are bound together so as to form a saturated or unsaturated, substituted or unsubstituted hydrocarbon chain comprising 4 or 5 members, optionally interrupted with one or several heteroatoms selected from N, S and 0,
∘ Rc is hydrogen, Br, Cl, I or F,
- R₂, R₃ and R₄ are defined as follows:
∘ either R₂=H and R₃ and R₄ are bound together and form a sequence -(CH₂)m- with m = 3, 4 or 5,
∘ or R₂, R₃ and R₄, either identical or different, represent a (C₁-C₄)alkyl group,
as a mixture of optical isomers or of diastereoisomers in any proportions, or as a form enriched with an optical isomer or with a diastereoisomer.

2. The peptidase substrate according to claim 1 **characterized in that** R₂, R₃ and R₄, either identical or different, represent a methyl or an ethyl.

3. The peptidase substrate according to claim 2 **characterized in that** R₂=R₃=R₄=-CH₃.

4. The peptidase substrate according to any one of claims 1 to 3 of formula (IA): 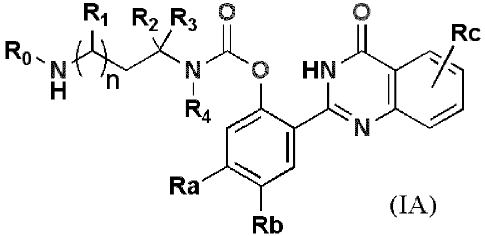 where R₀, R₁, R₂, R₃, R₄, n, Ra, Rb and Rc are as defined in claims 1 to 3, as a mixture of optical isomers or of diastereoisomers in any proportions, or as a form enriched with an optical isomer or with a diastereoisomer.

5. The peptidase substrate according to one of claims 1 to 4 **characterized in that** Ra=Rb=Rc=H or Ra=H, Rb=Rc=Cl.

6. The peptidase substrate according to one of claims 1 to 4 **characterized in that** Ra and Rb are bound together, so that the substrate according to the invention fits formula (IA') or (IB') : 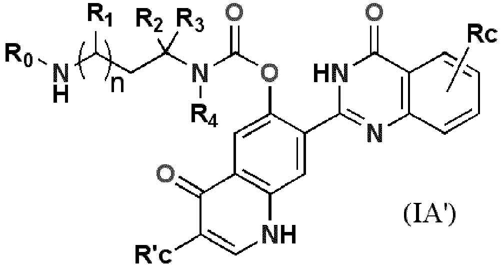 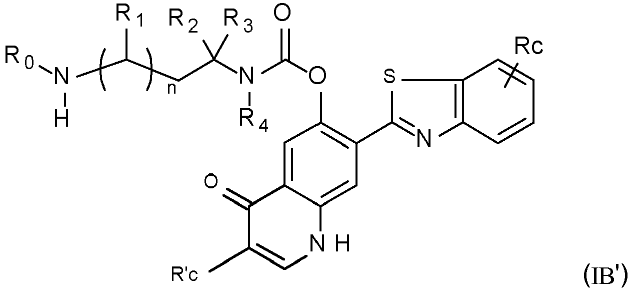 where n, R₀, R₁, R₂, R₃, R₄ and Rc are as defined in claims 1 to 3 and R'c represents a hydrogen atom or a -COO(C₁-C₄)alkyl group, as a mixture of optical isomers or of diastereoisomers in any proportions, or as a form enriched with an optical isomer or with a diastereoisomer.

7. The peptidase substrate according to any one of claims 1 to 6 **characterized in that** R₀ represents an amino acid or a peptidyl group including at most 10 amino acids in which the amino acids are either identical or different and selected from natural amino acids, and the N-terminal end may be unsubstituted or substituted with an acyl group -COR", R" being a (C₁-C₆)alkyl group or an O-(C₁-C₆) alkyl group.

8. The peptidase substrate according to any one of claims 1 to 7 **characterized in that** n=0.

9. The peptidase substrate according to any one of claims 1 to 8 **characterized in that** n=1 and R₁ represents a methyl, iso-propyl, iso-butyl or benzyl group.

10. The peptidase substrate according to any one of claims 1 to 9 **characterized in that** the peptidyl or amino acid group R₀ is a substrate for leucine aminopeptidase, caspase-3, HIV-1 peptidase, renin, thrombin, tryptase, cathepsin K, ACE plasmepsins I or II, β-secretase or prostate specific antigen "PSA".

11. The peptidase substrate according to claim 1 selected from the compounds of formula:

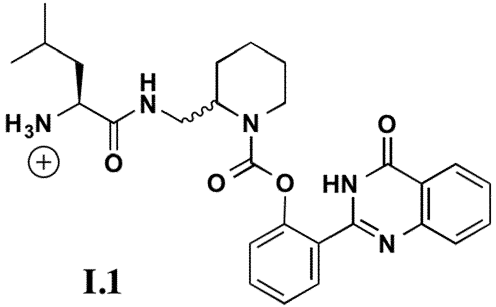

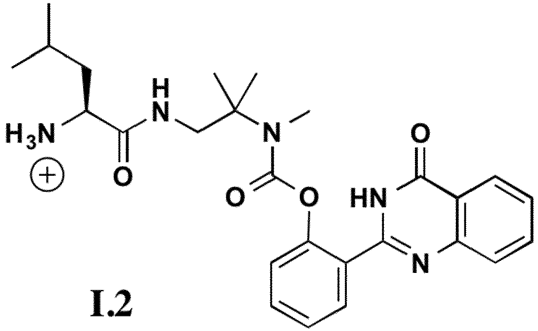

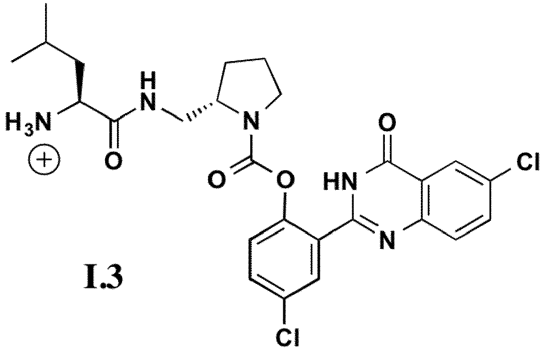

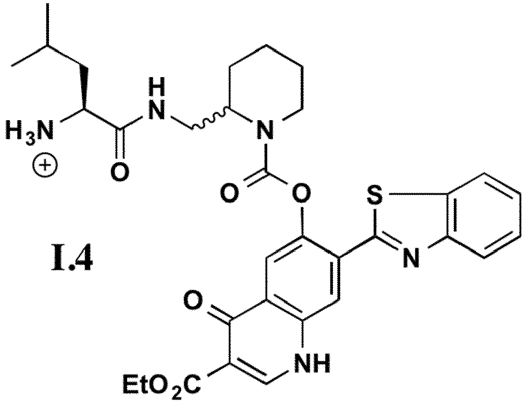

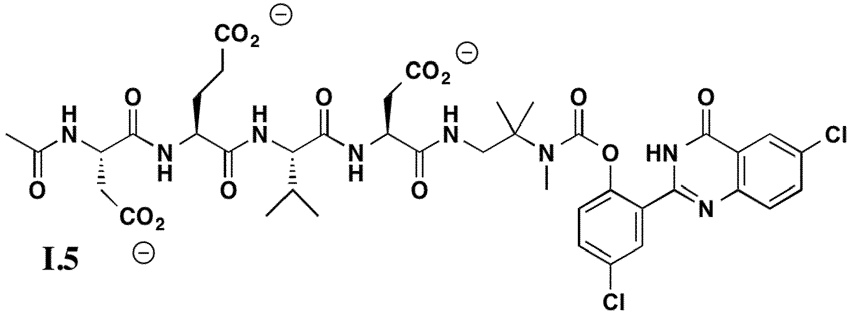

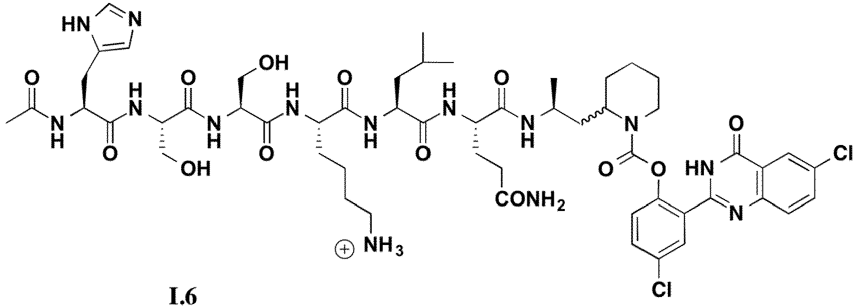

12. A method for detecting the presence of a catalytically active peptidase comprising the steps of:
- putting a sample suspected of containing said peptidase in contact with a substrate according to any one of claims 1 to 11;
- applying suitable conditions for allowing the formation of a fluorescent precipitate by cleavage of the covalent bond between NH and R₀, followed by cleavage of the -C(O)-R₅ bond following cyclization of the spacer; and
- quantitatively or qualitatively analysing said fluorescent precipitate.

13. The method according to claim 12 where the analysis of the fluorescent precipitate comprises the steps of:
- exposing the fluorescent precipitate to a light source capable of producing light at an absorption wavelength of the fluorescent precipitate; and
- detecting the fluorescence of the resultant precipitate.
